# EUROPÄISCHE PATENTSCHRIFT

(11) **EP 3 817 798 B1**
(45) Veröffentlichungstag und Bekanntmachung des Hinweises auf die Patenterteilung: **16.10.2024**
(21) Anmeldenummer: 19752076.0
(22) Anmeldetag: 05.07.2019
(51) Int. Cl.: A61M 16/00, A61M 16/08, A61M 16/20, A61M 16/12

(54) **UMBAUFREIE GASSTEUERUNGSVORRICHTUNG FÜR EIN BEATMUNGSGERÄT**
ALTERATION-FREE GAS CONTROL DEVICE FOR A VENTILATOR
DISPOSITIF DE COMMANDE DE GAZ SANS CONVERSION DESTINÉ À UN APPAREIL DE VENTILATION

(30) Priorität: 06.07.2018 DE 102018005340
(43) Veröffentlichungstag der Anmeldung: 12.05.2021
(73) Patentinhaber: Löwenstein Medical Technology S.A., 2557 Luxembourg (LU)
(72) Erfinder: ADAMETZ, Benjamin, 22609 Hamburg (DE)
(74) Vertreter: Marx, Thomas
(86) Internationale Anmeldenummer: PCT/DE2019/000185
(87) Internationale Veröffentlichungsnummer: WO 2020/007388

(56) Entgegenhaltungen:
- CN-A- 105 617 527
- CN-A- 107 308 531
- DE-A1- 102004 063 158
- GB-A- 1 190 441

## Beschreibung

Die vorliegende Erfindung betrifft eine Gassteuerungsvorrichtung, wobei die Gassteuerungsvorrichtung in einem Beatmungsgerät angeordnet ist, welches eingerichtet ist, eine Gaszu- und -abfuhr zu einem Patienten bereitzustellen, wobei die Gassteuerungsvorrichtung einen ersten Gaskanal mit einem Rückschlagventil, einen zweiten Gaskanal und eine Schalteinrichtung umfasst, wobei der erste Gaskanal eine Öffnung zum Ausströmen von Gas mit einer Dichtmembran und einer Verschlusskappe aufweist.

Beatmungsgeräte werden für die Therapie respiratorischer Störungen eingesetzt, dabei können die Beatmungsgeräte in der nicht-invasiven und invasiven Beatmung, sowohl innerklinisch als auch außerklinisch, verwendet werden.

Bei einer Beatmung eines Patienten kann in der Regel ein Beatmungsgerät mit einem inspiratorischen Zweig für den inspiratorischen Atemgasfluss und optional mit einem Zweig für den exspiratorischen Atemgasfluss eingesetzt werden. Der Zweig für den exspiratorischen Atemgasfluss ermöglicht die Ausatmung/Exspiration eines Atemgases durch den Patienten, während der Zweig für den inspiratorischen Atemgasfluss den Patienten mit Atemgas versorgt.

Die Beatmungsgeräte können im Betrieb mit einem Schlauchsystem mit passiver Ausatemöffnung/Leckageschlauchsystem oder einem Schlauchsystem mit aktivem Ausatemventil/Ventilschlauchsystem verbunden sein.

Bei im Stand der Technik bekannten Beatmungsgeräten ist es möglich, zwischen einer Beatmung mit einem Leckageschlauchsystem und einem Ventilschlauchsystem zu wechseln. Hierfür ist es bisher jedoch notwendig, extern oder intern des Gerätes einen Umbau/Einbau einer oder mehrerer Komponenten zum Beispiel eines Rückschlagventils vorzunehmen. Ein Leckageschlauchsystem ist dabei ein Einschlauchsystem mit definierter Leckageöffnung, durch die während der Beatmung fortlaufend Atemgas entweichen kann, um Kohlendioxid auszuspülen. Ein Ventilschlauchsystem ist ein Einschlauchsystem mit einem Schaltventil für die Exspiration oder ein Doppelschlauchsystem, bei dem ausgeatmetes Atemgas wieder dem Beatmungsgerät zur Überwachung zugeführt wird. Beatmungsgeräte weisen daher zumindest zwei Anschlussstutzen auf für entweder das Doppelschlauchsystem oder das Einschlauchsystem. Zusätzlich weisen Beatmungsgeräte Druckstutzen auf, die für die Verwendung von Einschlauchsystemen mit Ventil notwendig sind, um einen Steuerdruck auf das Ventil zu führen. Bei im Stand der Technik bekannten Beatmungsgeräten muss daher immer ein passender Adapter für das ausgewählte Schlauchsystem verwendet werden und zudem müssen ggfs. Druckstutzen verschlossen oder geöffnet werden. Bevor das Patientenschlauchsystem verbunden werden kann, muss der passende Schlauchsystemadapter installiert werden. Der Einbau/Umbau ist jedoch zeitaufwendig, fehleranfällig und bedeutet ein Anwendungshindernis.

CN 107 308 531 A beschreibt ein Beispiel für eine Gassteuerungsvorrichtung eines Beatmungsgeräts.

Die Erfindung ist durch die angefügten Ansprüche definiert.

Aufgabe der Erfindung ist es daher, eine Vorrichtung bereitzustellen, die es ermöglicht, ein Beatmungsgerät sowohl zusammen mit einem Leckageschlauchsystem als auch zusammen mit einem Ventilschlauchsystem ohne Umbaumaßnahmen oder ohne Adapter zu nutzen.

Diese Aufgabe wird durch ein Beatmungsgerät gemäß dem Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Erfindung betrifft ein Beatmungsgerät zur Verwendung mit alternativen Schlauchsystemen, wobei das Beatmungsgerät zumindest eine Atemgasquelle, zumindest einen Atemgasweg zumindest einen Geräteausgang und einen Geräteeingang umfasst und eine Gassteuervorrichtung zur Voreinstellung des Beatmungsgerätes für die Verwendung der alternativen Schlauchsysteme durch Schaltung von zumindest einer Schalteinrichtung, wobei die Gassteuerungsvorrichtung eingerichtet und ausgebildet ist für die Verwendung eines Schlauchsystems welches ein Leckagesystem ist den Atemgasweg (Bypass) für einen Atemgasstrom zu öffnen.

Das Beatmungsgerät ist auch dadurch gekennzeichnet, dass die Gassteuerungsvorrichtung eingerichtet und ausgebildet ist, die Schalteinrichtung zu aktivieren den Atemgasweg (Bypass) für einen Atemgasstrom zu öffnen und die Schalteinrichtung zu aktivieren den Anschluss zu schließen

Das Beatmungsgerät ist auch dadurch gekennzeichnet, dass die Gassteuerungsvorrichtung eingerichtet und ausgebildet ist, keinen Steuerdruck auf das Patientenventil und/oder zu leiten und den Flusssensor nicht zu aktivieren / zu nutzen.

Das Beatmungsgerät ist auch dadurch gekennzeichnet, dass die Gassteuerungsvorrichtung eingerichtet und ausgebildet ist, für die Verwendung eines Schlauchsystems welches ein Ventilsystem mit einem Patientenventil ist den Atemgasweg zu sperren und den Anschluss zu öffnen, um einen Steuerdruck auf das Patientenventil zu schalten.

Das Beatmungsgerät ist auch dadurch gekennzeichnet, dass die Gassteuerungsvorrichtung eingerichtet und ausgebildet ist, für die Verwendung eines Ventilsystems mit einem Patientenventil die Schalteinrichtung zu aktivieren einen Atemgasstrom durch den Atemgasweg zu sperren und die Schalteinrichtung zu aktivieren den Anschluss zu öffnen, um einen Steuerdruck auf das Patientenventil zu schalten.

Das Beatmungsgerät ist auch dadurch gekennzeichnet, dass die Gassteuerungsvorrichtung eingerichtet und ausgebildet ist, keinen Steuerdruck auf das Patientenventil zu leiten und den Flusssensor nicht zu aktivieren / zu nutzen.

Das Beatmungsgerät ist auch dadurch gekennzeichnet, dass die Gassteuerungsvorrichtung eingerichtet und ausgebildet ist, für die Verwendung eines Schlauchsystems welches ein Zweischlauchsystem ist den Atemgasweg zu sperren und den Anschluss zu sperren.

Das Beatmungsgerät ist auch dadurch gekennzeichnet, dass die Gassteuerungsvorrichtung eingerichtet und ausgebildet ist, die Schalteinrichtung zu aktivieren einen Atemgasstrom durch den Atemgasweg zu sperren und die Schalteinrichtung zu aktivieren den Anschluss zu sperren.

Das Beatmungsgerät ist auch dadurch gekennzeichnet, dass die Gassteuerungsvorrichtung eingerichtet und ausgebildet ist, einen Steuerdruck auf das Patientenventil zu leiten und der Flusssensor zu aktivieren.

Das Beatmungsgerät ist auch dadurch gekennzeichnet, dass die Gassteuerungsvorrichtung eingerichtet und ausgebildet ist, für die Verwendung eines:
Leckagesystems die Schalteinrichtung zu aktivieren den Atemgasweg (Bypass) für einen Atemgasstrom zu öffnen
Ventilsystems mit einem Patientenventil die Schalteinrichtung zu aktivieren einen Atemgasstrom durch den Atemgasweg zu sperren und die Schalteinrichtung aktiviert den Anschluss zu öffnen, um einen Steuerdruck auf das Patientenventil zu schalten Zweischlauchsystems die Schalteinrichtung zu aktivieren einen Atemgasstrom durch den Atemgasweg zu sperren und die Schalteinrichtung zu aktivieren den Anschluss zu sperren.

Das Beatmungsgerät ist auch dadurch gekennzeichnet, dass die Gassteuervorrichtung pneumatisch und/oder elektronisch ausgeführt ist und entsprechend einer Anwendervorgabe, über eine Anwenderschnittstelle, oder automatisch, wenn die Schlauchsysteme aufgrund technischer Kennungen von einem Erkennungssystem des Beatmungsgerätes erkannt werden, eine Voreinstellung des Beatmungsgerätes für die Verwendung der alternativen Schlauchsysteme vornimmt.

Die Gassteuervorrichtung umfasst zumindest das Rückschlagventil, den Bypass und das Ventil.

Die Gassteuervorrichtung umfasst alle Ventile und Schalteinrichtungen und Atemgasquellen.

Das Beatmungsgerät ist auch dadurch gekennzeichnet, dass der Atemgasweg und der Geräteausgang und der Anschluss und die zumindest eine Schalteinrichtung umbaufrei und adapterlos eingerichtet und ausgebildet sind, sodass ein Wechsel der Schlauchsysteme ohne Adapter oder ohne Werkzeuge möglich ist.

Insbesondere verbleiben der Geräteeingang und der Geräteausgang umbaufrei am Beatmungsgerät. Für die Verwendung der unterschiedlichen Schlauchsysteme sind bei einem Wechsel derselben Adapter für den Geräteeingang und den Geräteausgang nicht notwendig. Insbesondere müssen Geräteeingang oder Geräteausgang oder die Stutzen nicht mit Stopfen oder Kappen verschlossen werden.

Das Beatmungsgerät ist auch dadurch gekennzeichnet, dass die Schalteinrichtung als Ventil ausgeführt ist, welches zumindest zeitweise den Rückatemdruck der Exspiration des Patienten als Steuerdruck auf das Ventil schaltet.

Das Beatmungsgerät ist auch dadurch gekennzeichnet, dass die Schalteinrichtung als Verschlussventil ausgeführt ist, welches den Steuerdruck, der aus der Atemgasquelle kommt, auf den Druckstutzen schaltet, wenn dieser mit dem Patientenventil des Einschlauchsystems verbunden ist.

Das Beatmungsgerät ist auch dadurch gekennzeichnet, dass der Drucksensor bei Verwendung eines Leckagesystems inaktiv geschaltet wird, kann jedoch auch aktiv bleiben, wenn das Schlauchsystem einen Druckmesspunkt aufweist.

Das Beatmungsgerät ist auch dadurch gekennzeichnet, dass der Flusssensor bei Verwendung eines Leckagesystems inaktiv geschaltet oder nicht verwendet wird.

Das Beatmungsgerät ist auch dadurch gekennzeichnet, dass der Flusssensor 32c bei Verwendung eines Einschlauchsystems inaktiv geschaltet wird.

Das Beatmungsgerät ist auch dadurch gekennzeichnet, dass die Gassteuerungsvorrichtung eingerichtet ist, die Gasführung bei einem Beatmungsgerät mit einem wirkverbundenem Leckageschlauchsystem und mit einem wirkverbundenem Ventilschlauchsystem sicherzustellen, wobei die Gassteuerungsvorrichtung bei einem Wechsel zwischen Leckageschlauchsystem und Ventilschlauchsystem umbaufrei in dem Beatmungsgerät ausgebildet ist.

Das Beatmungsgerät ist auch dadurch gekennzeichnet, dass das Beatmungsgerät einen Atemgasweg der sich zwischen einem Geräteeingang und einem Geräteausgang erstreckt umfasst, wobei der Atemgasweg einen Atemgasantrieb und eine Gassteuerungsvorrichtung mit einem Rückschlagventil, einem Bypass und einer Schalteinrichtung a umfasst, wobei der Atemgasantrieb eine inspiratorische Atemgasströmung von dem Geräteeingang durch das Rückschlagventil zu dem Geräteausgang führt dadurch gekennzeichnet, dass das Rückschlagventil eingerichtet und angeordnet ist eine Atemgasströmung (in Sperrrichtung des Rückschlagventils) in Richtung des Geräteeingangs zu verhindern und der Bypass eingerichtet und angeordnet ist eine Atemgasströmung zur Umgehung des Rückschlagventils zu ermöglichen und das Schalteinrichtung a eingerichtet und angeordnet ist eine Atemgasströmung durch den Bypass zur Umgehung des Rückschlagventils zumindest zeitweise zu ermöglichen oder zu unterbrechen.

Das Beatmungsgerät ist auch dadurch gekennzeichnet, dass die Atemgasströmung durch den Bypass entgegen der Sperrrichtung des Rückschlagventils verläuft.

Das Beatmungsgerät ist auch dadurch gekennzeichnet, dass das Schalteinrichtung a in dem Bypass angeordnet ist und der Bypass von dem Atemgasweg, vor dem Rückschlagventil abzweigt und hinter dem Rückschlagventil wieder in den Atemgasweg oder in den Geräteeingang mündet.

Das Beatmungsgerät ist auch dadurch gekennzeichnet, dass der Atemgasantrieb zwischen dem Geräteeingang und dem Rückschlagventil angeordnet ist.

Das Beatmungsgerät ist auch dadurch gekennzeichnet, dass der Atemgasantrieb zwischen Rückschlagventil und dem Geräteausgang angeordnet ist.

Das Beatmungsgerät ist auch dadurch gekennzeichnet, dass die Schalteinrichtung in dem Bypass angeordnet ist und der Bypass zwischen dem Atemgasantrieb und dem Rückschlagventil abzweigt und mündet zwischen dem Rückschlagventil und dem Geräteausgang wieder in den Atemgasweg mündet und wobei der Atemgasantrieb zwischen dem Geräteeingang und dem Rückschlagventil angeordnet ist und über die im Bypass angeordnete Schalteinrichtung Atemgas in Richtung des Geräteeingangs rückgeführt werden kann, wodurch das Rückschlagventil umgangen wird.

Das Beatmungsgerät ist auch dadurch gekennzeichnet, dass die Schalteinrichtung a in dem Bypass angeordnet ist und der Bypass zwischen dem Geräteeingang und dem Rückschlagventil abzweigt und zwischen dem Rückschlagventil und dem Atemgasantrieb wieder in den Atemgasweg mündet, wobei der Atemgasantrieb zwischen dem Rückschlagventil und dem Geräteausgang angeordnet ist und über die im Bypass angeordnete Schalteinrichtung Atemgas in Richtung des Geräteeingangs rückgeführt werden kann, nachdem es den Atemgasantrieb passiert hat.

Das Beatmungsgerät ist auch dadurch gekennzeichnet, dass ein zweiter Atemgasweg von einem Geräteeingang, zu dem Geräteausgang führt wobei der zweite Atemgasweg vor dem Geräteausgang und stromabwärts hinter dem Rückschlagventil und hinter dem Atemgasantrieb in den ersten Atemgasweg mündet.

Das Beatmungsgerät ist auch dadurch gekennzeichnet, dass in dem zweiten Atemgasweg ein Rückschlagventil und/oder ein Atemgasantrieb angeordnet ist, um einen definierten Atemgasstrom von dem Geräteeingang, zu dem Geräteausgang zu führen.

Das Beatmungsgerät ist auch dadurch gekennzeichnet, dass eine Gasmesseinrichtung zwischen dem Bypass und dem Geräteausgang angeordnet ist und eingerichtet ist einen Fluss oder ein Volumen oder einen Druck des Atemgases im Atemgasweg zu erfassen und die Schalteinrichtung a eingerichtet und ausgebildet ist einen PEEP (positive end-expiratory pressure) im Bereich von 0-20hPa, bevorzugt 0-15hPa einzustellen.

Das Beatmungsgerät ist auch dadurch gekennzeichnet, dass das Beatmungsgerät einen exspiratorischen Atemgasweg aufweist, der sich von einem exspiratorischen Geräteeingang zu einem exspiratorischen Geräteausgang erstreckt und eine Schalteinrichtung und eine Gasmesseinrichtung umfasst, wobei die Gasmesseinrichtung zwischen dem exspiratorischen Geräteausgang und der Schalteinrichtung angeordnet ist.

Das Beatmungsgerät ist auch dadurch gekennzeichnet, dass ein Schlauchsystem 33 an den Geräteausgang montiert ist, wobei das Schlauchsystem 33 einen ersten Zweig 34 und einem zweiten Zweig 35 aufweist, wobei der erste Zweig 34 von dem Geräteausgang zu einem Patienteninterface 36 führt und von dem Patienteninterface 36 ein zweiter Zweig 35 zu dem exspiratorischen Geräteeingang 38 führt, wobei über den ersten Zweig 34 inspiratorisches Atemgas aus dem inspiratorischen Atemgasweg zu dem Patienteninterface 36 geführt wird und über den zweiten Zweig 35 exspiratorisches Atemgas aus dem Patienteninterface 36 zu dem exspiratorischen Atemgasweg geführt wird.

Das Beatmungsgerät ist auch dadurch gekennzeichnet, dass bei einer Blockade/Störung des exspiratorischen Atemgasweges das exspiratorische Atemgas durch Steuerung der Schalteinrichtung in den inspiratorischen Atemgasweg und über den geöffneten Bypass in die Umgebung abgeführt werden kann.

Das Beatmungsgerät ist auch dadurch gekennzeichnet, dass das Beatmungsgerät einen ersten inspiratorischen Atemgasweg und einen zweiten inspiratorischen Atemgasweg sowie einen exspiratorischen Atemgasweg und einen separaten exspiratorischen Atemgasweg aufweist. Erfindungsgemäß ist die Gassteuerungsvorrichtung eingerichtet, das Gasführung bei einem Beatmungsgerät mit einem wirkverbundenem Leckageschlauchsystem und mit einem wirkverbundenem Ventilschlauchsystem sicherzustellen, wobei die Gassteuerungsvorrichtung, bei einem Wechsel zwischen Leckageschlauchsystem und Ventilschlauchsystem umbaufrei in dem Beatmungsgerät ausgebildet ist.

Dadurch ist die erfindungsgemäße Gassteuerungsvorrichtung sowohl für die Verwendung in einem mit einem Leckageschlauchsystem wirkverbundenem Beatmungsgerät als auch für die Verwendung in einem mit einem Ventilschlauchsystem wirkverbundenem Beatmungsgerät eingerichtet. Dabei zeichnet sich die vorliegende Erfindung dadurch aus, dass die Gassteuerungsvorrichtung umbaufrei ist. Umbaufrei bedeutet, dass zwischen dem Leckageschlauchsystem und dem Ventilschlauchsystem an einem Beatmungsgerät gewechselt werden kann, ohne dass es einer Umbaumaßnahme der Gassteuerungsvorrichtung im/am Beatmungsgerät bedarf. Dies bietet den Vorteil, dass die derart eingerichtete Gassteuerungsvorrichtung sowohl in einem Beatmungsgerät, welches im Leckageschlauchsystem betrieben wird, als auch in einem Beatmungsgerät, welches im Ventilschlauchsystem betrieben wird, benutzt werden kann.

Bei einem Wechsel von einem Leckageschlauchsystem/Leckagesystem zu einem Ventilschlauchsystem/Ventilsystem bedarf es der vorliegenden Gassteuerungsvorrichtung Umsteuerung von Gas in den Gaskanälen, um beispielsweise bei einem Leckageschlauchsystem Atemgas/Gas durch die Öffnung des ersten Gaskanals abzuführen, sodass es nicht zu einem Druckanstieg oder einer Blockade des Gasstroms im Inneren des Schlauchsystems kommt. Die erfindungsgemäße Gassteuerungsvorrichtung ist somit eingerichtet, das Gas in dem Beatmungsgerät bei Bedarf derart umzusteuern, dass das Gas über verschiedene Gaskanäle des Beatmungsgeräts führbar ist.

In einer alternativen Ausführungsform kann ein Beatmungsgerät mindestens zwei Gassteuerungsvorrichtungen umfassen, um einen Gasstrom in dem Beatmungsgerät optimal in Abhängigkeit eines gewählten Schlauchsystems oder einer auftretenden Blockade/Störung zu führen.

In Ausgestaltung umfasst die Gassteuerungsvorrichtung eine Schalteinrichtung, wobei die Schalteinrichtung eingerichtet ist, zwischen mindestens zwei Schaltungsmodi zu schalten, die das Beatmungsgerät für die Verwendung von zumindest zwei unterschiedlichen Schlauchsystemen einrichten. Die vorliegende Gassteuerungsvorrichtung umfasst mindestens eine Schalteinrichtung. Die Schaltungsmodi sind vorteilhafterweise in der Schalteinrichtung hinterlegt/gespeichert. Die Schaltungsmodi sind in der Regel in der Gassteuerungsvorrichtung / Schalteinrichtung hinterlegbar, wobei die Schaltungsmodi vorteilhafterweise einstellbar ausgebildet sind. Vorteilhafterweise ist die Gassteuerungsvorrichtung oder Schalteinrichtung eingerichtet, durch eine Sperrfunktion nur durch Fachpersonal einstellbar zu sein. Alternativ sind voreingestellte Schaltungsmodi hinterlegt. Die Schaltungsmodi können entsprechend einer Exspiration des Beatmungsgerätes oder entsprechend einer Inspiration geschaltet/gewechselt werden. Alternativ können die Schaltungsmodi manuell oder automatisch gewechselt werden.

In einer weiteren Ausgestaltung ist die Schalteinrichtung eingerichtet, bei der Verwendung eines Leckageschlauchsystems die Gassteuerungsvorrichtung in einen ersten Schaltungsmodus zu schalten und bei der Verwendung eines Ventilschlauchsystems in einen zweiten Schaltungsmodus zu schalten.

Der erste Schaltungsmodus zeichnet sich in der Regel dadurch aus, dass die Schalteinrichtung derart geschaltet wird, dass bei einem Wechsel zu einem Leckageschlauchsystem der Gasstrom des ersten Gaskanals über die Öffnung des ersten Gaskanals kontinuierlich abführbar ist. Hierfür ist die Schalteinrichtung eingerichtet, beim Einstellen des ersten Schaltungsmodus den Gasstrom des zweiten Gaskanals nicht mit einem Arbeitsdruck zu beaufschlagen, sodass eine Dichtmembran, die auf der Öffnung aufliegt, durch den Gasstrom des ersten Gaskanals abhebbar ist und das Gas/der Gasstrom des ersten Gaskanals über die Öffnung entweichen kann. Vorteilhafterweise liegt der erste Schaltungsmodus bei einer Verwendung der Gassteuerungsvorrichtung in einem Leckageschlauchsystem dauerhaft an. Dadurch ist ein kontinuierlicher Abfluss von Gas auf der Öffnung garantiert, so dass es nicht zu einem Druckanstieg oder einer Blockade in dem ersten Gaskanal des Beatmungsgerätes kommt.

Der zweite Schaltungsmodus zeichnet sich in der Regel dadurch aus, dass die Schalteinrichtung derart geschaltet wird, dass bei einem Wechsel zu einem Ventilschlauchsystem die Schalteinrichtung den Gasstrom des zweiten Gaskanals mit einem Arbeitsdruck beaufschlagt, so dass der beaufschlagte Gasstrom des zweiten Gaskanals die Dichtmembran auf der Öffnung abdichtet, sodass der Gasstrom des ersten Gaskanals nicht über die Öffnung entweichen kann. Dies bietet den Vorteil, dass keine Rückatmung in das Beatmungsgerät in der Exspiration erfolgt, die die COz- Abführung des Atemgases verschlechtern würde. Der zweite Gaskanal ist somit in der Regel als Druckkanal ausgebildet.

In einer Weiterbildung der Erfindung ist die Schalteinrichtung eingerichtet, bei einer Blockade eines exspiratorischen Zweiges des Beatmungsgerätes in einen dritten Schaltungsmodus zu schalten. Dabei ist der dritte Modus ähnlich dem ersten Schaltungsmodus eingerichtet, wobei der Gasstrom des ersten Gaskanals über die Öffnung entweichen kann. Der dritte Schaltungsmodus ist beispielsweise anwendbar, wenn ein Atemgasantrieb/ein Gebläse ausfällt. In diesem Fall kann über den dritten Schaltungsmodus Gas aus der Umgebung durch den Patienten angesaugt werden. In der Regel ist der dritte Schaltungsmodus eingerichtet, durch eine Störung/Blockade des Gasstroms in dem ersten Gaskanal ausführbar zu sein. Das bedeutet, dass die Schalteinrichtung vorteilhafterweise eingerichtet ist, eine Störung/Blockade in dem ersten Gaskanal zu erfassen/zu erkennen. Hierfür kann die Schalteinrichtung eine Messvorrichtung umfassen, die eingerichtet sein kann, einen Druck, ein Volumen und/oder eine Durchflussrate des Gasstroms des ersten Gaskanals zu erfassen. Die Schalteinrichtung kann eingerichtet sein, basierend auf einem erfassten Anstieg eines Drucks oder eines Volumens über einen vorbestimmten Zeitraum automatisch zu schalten. Dadurch wird bei einer Blockade/Störung des ersten Gaskanals automatisch eine Abführung von Gas/Atemgas garantiert. Optional kann die Schalteinrichtung kontinuierlich stufenlos schaltbar sein. Dies bietet den Vorteil, dass die Öffnung der Umgehung des Rückschlagventils in eine Zwischenstellung steuerbar ist.

In einer weiteren Weiterbildung sind die Schaltungsmodi über die Schalteinrichtung wählbar und einstellbar. In der Regel sind mindestens zwei Schaltungsmodi in der Schalteinrichtung voreingestellt. Die Schaltungsmodi können optional über eine Sperrfunktion der Schalteinrichtung durch Fachpersonal einstellbar bzw. wählbar sein. Dadurch kann behandelndes Fachpersonal auch außerhalb der voreingestellten Schaltungsmodi die Schalteinrichtung steuern oder für einen Patienten einen angepassten Schaltungsmodus einrichten und speichern.

In Ausgestaltung umfasst die Gassteuerungsvorrichtung einen ersten Gaskanal und einen zweiten Gaskanal, wobei der Gasstrom aus dem ersten Gaskanal abführbar ist. Der erste Gaskanal ist in der Regel als Inspirationszweig eingerichtet, der ein Atemgas einem Patienten zuführt. Bei einem Leckageschlauchsystem wird der erste Gaskanal jedoch auch von rückströmender AtemGas des Patienten erreicht, die abgeführt werden muss. Durch die Schaltung der Schalteinrichtung in den ersten Schaltungsmodus kann das Gas aus dem ersten Gaskanal über die Öffnung abgeführt werden. Bei einem Ventilschlauchsystem ist der erste Gaskanal durch das Rückschlagventil in der Exspiration geschlossen. Hierfür wird in der Regel der zweite Gaskanal mit einem Arbeitsdruck beaufschlagt, so dass die Dichtmembran auf die Öffnung gedrückt wird, und das Gas des ersten Gaskanals nicht über die Öffnung entweichen kann, sondern dem schlauchsysteminternen Ventil zuführbar ist. Optional ist das schlauchsysteminterne Ventil dem Rückschlagventil vorgeschaltet und somit auf der Druckseite des Beatmungsgerätes ausgebildet.

In einer weiteren Ausgestaltung ist die Schalteinrichtung eingerichtet, in dem ersten Schaltungsmodus die Gassteuerungsvorrichtung derart zu steuern, dass der Gasstrom über eine Öffnung in dem ersten Gaskanal, auf der eine Dichtmembran aufliegt, an eine Ansaugseite des Gebläses führbar ist. Vorteilhafterweise wird das Gas in dem ersten Gaskanal somit patientenfern abgeführt. In der Regel ist das durch die Öffnung abgeführte Gas in einem Umgehungskanal zu der Ansaugseite führbar. Alternativ kann das abgeführte Gas über den Umgehungskanal wieder dem ersten Gaskanal zuführbar ausgebildet sein.

In einer Weiterbildung der Erfindung ist die Schalteinrichtung in dem ersten Schaltungsmodus eingerichtet, den Gasstrom des ersten Gaskanals an einem Rückschlagventil vorbeizuführen. In der Regel weist ein Beatmungsgerät bei der Verwendung eines Ventilschlauchsystems ein Rückschlagventil zum Schutz des Beatmungsgerätes vor zurückgeführter kontaminierter Ausatemgas des Patienten auf. Nachteilig an derartigen Rückschlagventilen ist, dass diese bei einer Blockade/Störung des aktiven Ausatmenventils einen Patienten an einer Ausatmung hindern bzw. diese vollständig unterbinden. Daher ist die Schalteinrichtung vorteilhafterweise derart in dem Beatmungsgerät angeordnet, dass mittels der Ausführung von Gas aus der Öffnung des ersten Gaskanals in den Umgehungsweg ein Rückschlagventil umgehbar ist.

In einer weiteren Weiterbildung ist die Schalteinrichtung eingerichtet, die Gassteuerungsvorrichtung in dem zweiten Schaltungsmodus derart zu steuern, dass die Dichtmembran mit einem Arbeitsdruck des Patienten beaufschlagt wird. In der Regel ist der erste Gaskanal mit dem zweiten Gaskanal verbunden ausgebildet, wobei der zweite Gaskanal mit einem Arbeitsdruck beaufschlagbar ist. Der Arbeitsdruck wird in der Regel durch die Schalteinrichtung bereitgestellt.

Die Schalteinrichtung kann auch variabel schaltbar eingerichtet sein, wobei die Schalteinrichtung eingerichtet sein kann, einen Druck des Gasstroms in dem zweiten Gaskanal zwischen 0mbar und 1bar, insbesondere zwischen 0mbar und 100mbar stufenlos zu steuern. Die Schalteinrichtung kann auch eingerichtet sein, den Gasstrom/Druck in dem zweiten Gaskanal kontinuierlich über einen vorbestimmten Zeitraum auf einen vorbestimmten Wert zu erhöhen.

In Ausgestaltung ist die Schalteinrichtung eingerichtet, bei einem Wechsel eines Schlauchsystems bei Bedarf den Schaltungsmodus zu wechseln. In der Regel ist die Gassteuerungsvorrichtung eingerichtet einen Schlauchsystemwechsel zu erkennen, wobei die Schalteinrichtung der Gassteuerungsvorrichtung eingerichtet ist, basierend auf dem erkannten Schlauchsystem in den entsprechenden Schaltungsmodus zu schalten. In der Regel erfolgt die Bestimmung des Schlauchsystems über eine Auswertung von durch einen Sensor der Schalteinrichtung erfassten Atemgasparametern. Alternativ kann die Erkennung des verwendeten Schlauchsystems manuell über ein User-Interface des Beatmungsgerätes durch den Patienten oder bedienendes Fachpersonal erfolgen.

In weiterer Ausgestaltung ist die Gassteuerungsvorrichtung in Inspirationsflussrichtung dem Gebläse vorgeschaltet angeordnet. Dies bietet den Vorteil, dass die Gassteuerungsvorrichtung mit der Schalteinrichtung auf der Ansaugseite des Beatmungsgerätes angeordnet ist. Alternativ kann die Gassteuerungsvorrichtung auf der Druckseite des Beatmungsgerätes, also in Inspirationsflussrichtung dem Gebläse nachgeschaltet angeordnet sein. Dies bietet eine alternative Anordnungsmöglichkeit, was zu einer größeren Gestaltungsfreiraum bei der Anordnung der Gassteuerungsvorrichtung in dem Beatmungsgerät beiträgt.

Die vorliegende Erfindung umfasst des Weiteren ein Beatmungsgerät umfassend eine Gassteuerungsvorrichtung gemäß mindestens einem der vorgenannten Merkmale.

Gas kann im Sinne der Erfindung jedes atembare Gas oder Gasgemisch sein. Insbesondere Luft, Sauerstoff, Atemgas der Exspiration oder Atemgas der Inspiration.

Im Folgenden werden anhand von stark vereinfachten schematischen Darstellungen bevorzugte Ausführungsbeispiele der Erfindung näher erläutert. Es zeigt:
Fig. 1 eine schematische Ansicht einer erfindungsgemäßen Gassteuerungsvorrichtung, wobei die Gassteuerungsvorrichtung hier nur teilweise dargestellt nämlich als Schalteinrichtung oder als Ventilblock
Fig. 2 eine schematische Explosionsansicht der in Figur 1 gezeigten Schalteinrichtung/Ventilblock
Fig. 3 einen Längsschnitt durch die in den Figuren 1 und 2 gezeigte Schalteinrichtung/Ventilblock,
Fig. 4a eine Seitenansicht der in den Figuren 1 bis 3 gezeigten Schalteinrichtung/Ventilblock,
Fig. 4b eine Draufsicht auf eine erfindungsgemäße Verschlusskappe der in den Figuren 1 bis 4a gezeigten Schalteinrichtung/Ventilblock,
Fig. 4c eine Seitenansicht auf ein Rückschlagventil der in den Figuren 1 bis 4b gezeigten Schalteinrichtung/Ventilblock,
Fig. 5 zeigt eine Ausführungsform eines schematischen Aufbaus einer pneumatischen Schaltanordnung der erfindungsgemäßen Gassteuerungsvorrichtung,
Fig. 6 zeigt eine Ausführungsform eines schematischen Aufbaus einer weiteren pneumatischen Schaltanordnung der erfindungsgemäßen Gassteuerungsvorrichtung,
Fig. 7 zeigt eine Ausführungsform eines schematischen Aufbaus einer weiteren pneumatischen Schaltanordnung der erfindungsgemäßen Gassteuerungsvorrichtung,
Fig. 8 zeigt eine Ausführungsform eines schematischen Aufbaus einer weiteren pneumatischen Schaltanordnung der erfindungsgemäßen Gassteuerungsvorrichtung,
Fig. 9 zeigt eine Ausführungsform eines schematischen Aufbaus einer weiteren pneumatischen Schaltanordnung der erfindungsgemäßen Gassteuerungsvorrichtung,
Fig. 10 zeigt eine Ausführungsform eines schematischen Aufbaus einer weiteren pneumatischen Schaltanordnung der erfindungsgemäßen Gassteuerungsvorrichtung,
Fig. 11 zeigt eine Ausführungsform eines schematischen Aufbaus einer weiteren pneumatischen Schaltanordnung der erfindungsgemäßen Gassteuerungsvorrichtung,
Fig. 12 zeigt eine Ausführungsform eines schematischen Aufbaus einer weiteren pneumatischen Schaltanordnung der erfindungsgemäßen Gassteuerungsvorrichtung,
Fig. 13 - 15 zeigen je eine Ausführungsform der erfindungsgemäßen Gassteuerungsvorrichtung bei Verwendung von unterschiedlichen Schlauchsystemen.

In den Figuren weisen dieselben konstruktiven Elemente jeweils dieselben Bezugsziffern auf.

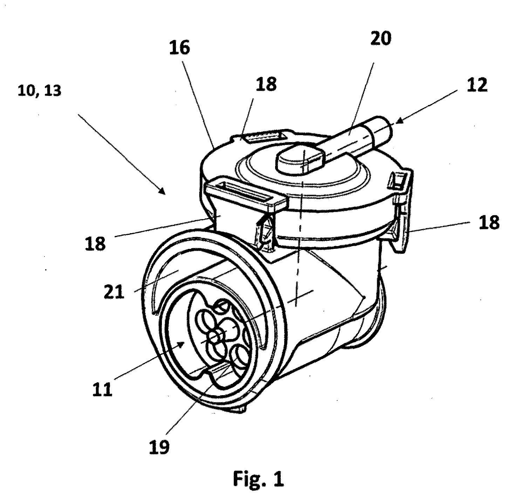

Figur 1 zeigt eine schematische Ansicht einer erfindungsgemäßen Gassteuerungsvorrichtung 10 für ein Beatmungsgerät, wobei hier lediglich ein Ventilblock oder eine Schalteinrichtung 13 dargestellt ist, der Teil der Gassteuerungsvorrichtung ist.

Die in der Figur 1 gezeigte erfindungsgemäße Schalteinrichtung 13 ist zur Verwendung in einem Beatmungsgerät zur Verwendung mit einem Leckageschlauchsystem und/oder einem Ventilschlauchsystem und/oder Zweischlauchsystem vorgesehen und weist einen ersten Gaskanal 11 (inspiratorischen Zweig) auf, der einen Gasstrom in Richtung eines Patienten führt. Der erste Gaskanal 11 umfasst ein Rückschlagventil 19, sowie eine - nicht gezeigte - Öffnung mit einer - nicht gezeigten - Dichtmembran. Die Öffnung und die Dichtmembran sind in der Figur 1 von einer Verschlusskappe 16 verdeckt. Das Rückschlagventil 19 verhindert eine Rückführung eines von dem Patienten kommenden Atemgases/Gässtroms in Richtung des Beatmungsgerätes.

Zudem weist die Schalteinrichtung 13 einen zweiten Gaskanal 12 auf, der einen Druck auf die - nicht gezeigte - Dichtmembran auf die - nicht gezeigte - Öffnung führt. Der zweite Gaskanal 12 ist über einen Anschluss 20 mit einer Verschlusskappe 16 verbunden, die auf die Öffnung des ersten Gaskanals 11 aufgeklippt ist. Der zweite Gaskanal 12 verläuft über den Anschluss 20 zu der Verschlusskappe 16 und wird durch eine - nicht gezeigte - Schalteinrichtung gesteuert. Der zweite Gaskanal 12 kann von einem Gasstrom durchflossen werden.

Die Schalteinrichtung kann den zweiten Gaskanal 12 mit einem Arbeitsdruck beaufschlagen oder beaufschlagungsfrei halten. Bei einem vorbestimmten Betriebszustand des Beatmungsgerätes ist der zweite Gaskanal 12 mit einem Arbeitsdruck beaufschlagt, so dass der beaufschlagte Gasstrom bzw. Druck über den Anschluss 20 auf die - nicht gezeigte - Dichtmembran innerhalb der Verschlusskappe 16 geführt wird. Durch den beaufschlagten Gasstrom/Druck des zweiten Gaskanals 12 wird die Dichtmembran vollständig auf die Öffnung gedrückt und die Öffnung somit verschlossen.

In der Figur 1 ist ferner der Umgehungskanal 21 dargestellt. Der Umgehungskanal 21 erstreckt sich von der Öffnung und ermöglicht die Vorbeiführung des aus der Öffnung ausströmenden Gasstroms an dem Rückschlagventil 19 vorbei. Der Umgehungskanal 21 ist umlaufend um die Öffnung ausgebildet und verengt sich in seinem weiteren Verlauf zu einem Kanal, der sich in einer Richtung, parallel zu dem ersten Gaskanal 11 erstreckt. Der Umgehungskanal 21 kann anschließend erneut in den ersten Gaskanal 11 einmünden oder als separater Zweig den an dem Rückschlagventil 19 vorbeigeführten Gasstrom abführen. Der Umgehungskanal 21 weist im Bereich der - nicht gezeigten - Öffnung einen Rand auf, auf den die Dichtmembran durch die Verschlusskappe 16 abgedichtet werden kann. Der Rand des Umgehungskanals 21 kann eine Hervorhebung aufweisen, hinter die an eine Kante der Dichtmembran eingreifen kann. Der Rand des Umgehungskanals 21 verhindert ein Verrutschen der Dichtmembran.

Zudem ist in der Figur 1 die Verschlusskappe 16 dargestellt. Die Verschlusskappe 16 umfasst in der vorliegenden Ausführungsform drei Fortsätze 18 mittels derer die Verschlusskappe 16 auf die Öffnung des ersten Gaskanals 11 aufklippbar ist. Zudem ist an der Verschlusskappe 16 ein Anschluss 20 ausgebildet, mit dem der zweite Gaskanal 12 verbindbar ist. Der Anschluss 20 ist mittig auf der Verschlusskappe 16 angeordnet, so dass der Gasstrom/Druck über den Anschluss 20 und die Verschlusskappe 16 mittig und gleichmäßig auf die Dichtmembran aufgebracht werden kann.

Die vorstehend beschriebene Schalteinrichtung wird durch die Gassteuerungsvorrichtung 10 geschaltet. In der Regel schaltet die Schalteinrichtung den zweiten Gaskanal 12 beaufschlagungsfrei sobald das Beatmungsgerät auf Exspiration umschaltet. In diesem Fall wird kein Arbeitsdruck auf den Gasstrom des zweiten Gaskanals 12 aufgegeben. Im Gegenzug schaltet die Schalteinrichtung den zweiten Gaskanal 12 arbeitsdruckfrei/beaufschlagungsfrei, wenn das Beatmungsgerät auf Inspiration schaltet.

Die vorstehend beschriebe Gassteuerungsvorrichtung kann für verschiedene Verwendungen eingesetzt werden.

Bei einem Leckageschlauchsystem ist die Schalteinrichtung eingerichtet, den Gasstrom des zweiten Gaskanals 12 beaufschlagungsfrei zu schalten. In diesem Fall ist das Schaltventil eingerichtet dauerhaft ein Ausströmen von Gas zu ermöglichen.

Bei einem Ventilschlauchsystem ist die Schalteinrichtung eingerichtet, entsprechend der zeitgesteuerten Exspiration des Beatmungsgerätes zu schalten. Somit kann die Schalteinrichtung beispielsweise bei der Schaltung des Beatmungsgerätes auf eine Exspiration oder bei einer Blockade des exspiratorischen Zweiges, den zweiten Gaskanal 12 beaufschlagunsgfrei schalten. Dadurch wird die Exspiration eines Patienten sichergestellt, da die Öffnung der Gassteuerungsvorrichtung 10 während der Exspiration geöffnet ist und eine rückströmende Gas über die Öffnung entweichen kann.

Bei einem Betrieb im Ventilschlauchsystem schaltet die Schalteinrichtung den zweiten Gaskanal 12 mit dem Arbeitsdruck. Durch den beaufschlagten Druck in dem zweiten Gaskanal 12 wird die Dichtmembran auf der Öffnung abgedichtet, wodurch ein Ausströmen von inspiratorischer Gas durch die Öffnung während dieser Betriebsart verhindert wird.

Im Allgemeinen ist die Gassteuerungsvorrichtung 10 bei der Inspiration eingerichtet, den Gasstrom in dem ersten Gaskanal 11 durch das Rückschlagventil 19 zu dem Patienten hin zu führen. In der Exspiration wird die Öffnung des ersten Gaskanals 11 somit verschlossen und die Funktion des Rückschlagventils 19 bleibt bestehen.

Bei einer Blockade/Störung eines exspiratorischen Zweiges des Beatmungsgerätes hingegen wird ein Ausatmen des Patienten verhindert oder erschwert. Durch das Rückschlagventil 19 kann der Patient zudem nicht über den inspiratorischen Zweig ausatmen. Bei einer Blockade/Störung des exspiratorischen Zweiges des Beatmungsgerätes ist die Schalteinrichtung daher eingerichtet, den zweiten Gaskanal 12 beaufschlagunsgfrei zu schalten. Dadurch, dass der zweite Gaskanal 12 nicht mit einem Arbeitsdruck beaufschlagt wird, der auch auf die Dichtmembran wirkt, kann der Gasstrom des ersten Gaskanals 11 die Dichtmembran, die auf der Öffnung aufliegt, anheben. Dadurch kann der Gasstrom des ersten Gaskanals 11 über einen Umgehungskanal 21 um das Rückschlagventil 19 herumgeführt werden.

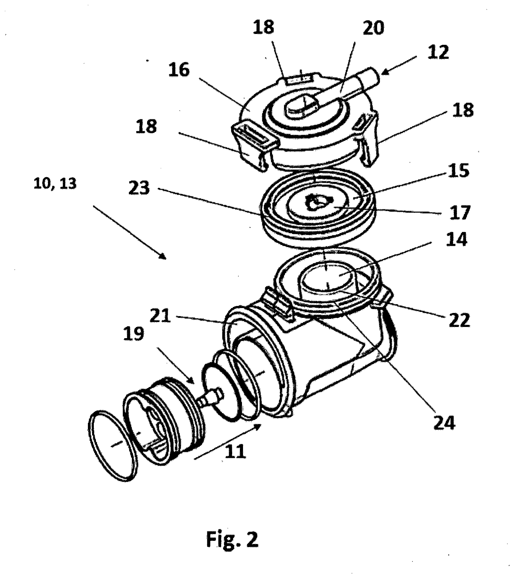

Figur 2 zeigt eine schematische Explosionsansicht der in Figur 1 gezeigten Schalteinrichtung 13. Dabei sind der erste und der zweite Gaskanal 11, 12, das Rückschlagventil 19 sowie die Öffnung 14 mit der Dichtmembran 15 gezeigt.

Dargestellt ist auch die Verschlusskappe 16 mit dem Anschluss 20. Gezeigt ist die mittige Anordnung des Anschlusses 20, wobei ein Ende des Anschlusses 20 sich nach außen erstreckt und mit dem zweiten Gaskanal 12 verbindbar ist.

In der Figur 2 ist gezeigt, dass die Öffnung 14 einen Rand 22 aufweist. Der Rand 22 ist als Erhebung ausgebildet und dient als Ablage für das in die Dichtmembran 15 integriert ausgebildete Gewicht 17.

Die Dichtmembran 15 ist aus einem elastomeren Material, vorzugsweise einem Silikon mit einer Härte zwischen 15 bis 25 Shore A, insbesondere zwischen 18 und 22 Shore A ausgebildet. Die Dichtmembran 15 ist kreisförmig ausgebildet und weist eine Begrenzung 23 auf.

Die Begrenzung 23 kann als eine verstärkte Struktur oder als funktionale Hervorhebung ausgebildet sein. Die Begrenzung 23 ist eingerichtet, auf einem Rand 24 des Umgehungskanals 21 aufzuliegen. Die Dichtmembran 15 kann zwischen der Begrenzung 23 der Dichtmembran 15 und dem Gewicht 17 eine dünnere Materialstärke aufweisen als im Bereich des Randes 24 des Umgehungskanals 21 und des Bereichs der Dichtmembran 15, in dem das Gewicht 17, zumeist integral, angeordnet ist.

In der vorliegenden Ausführungsform weist die Dichtmembran 15 zwischen dem Rand 24 des Umgehungskanals 21 und dem Gewicht 17 eine gewölbte Struktur auf, die zusätzlich einer Verschiebung der Dichtmembran 15 auf der Öffnung 14 vorbeugen kann. In weiteren Ausführungsformen kann die Dichtmembran 15 zwischen dem Rand 24 des Umgehungskanals 21 und dem Gewicht 17 eine andere Struktur aufweisen oder strukturfrei ausgebildet sein.

Das Gewicht 17 der Dichtmembran 15 ist in Form einer Unterlegscheibe ausgebildet. Das Gewicht 17 ist aus einem Metall gebildet und in die Dichtmembran 17 integral ausgebildet. Das Gewicht 17 kann die Dichtmembran 15 auf der Öffnung 14 stabilisieren. In der Regel dient das Gewicht 17 dazu, Schwingungen der Dichtmembran 15 durch aus dem ersten Gaskanal ausströmende Gas zu verhindern.

Die in der Figur 2 gezeigte Verschlusskappe 16 umfasst Fortsätze 18, die Widerhaken ausbilden. Die Widerhaken greifen beim Aufklippen der Verschlusskappe 16 in Ausnehmungen ein, die an der Außenseite der Gassteuerungsvorrichtung 10 ausgebildet sind. In weiteren Ausführungsformen sind weitere Verschlussmöglichkeiten, beispielsweise eine Verrastung in Form eines Bajonettverschlusses denkbar.

Beim Aufsetzen der Verschlusskappe 16 auf die Öffnung 14 wird die Dichtmembran 15 im Bereich der Begrenzung 23 der Dichtmembran 15 an den Rand des Umgehungskanals 21 angedrückt und gehalten. Die Verschlusskappe 16 dichtet die Dichtmembran 15 nach außen ab und hält die Dichtmembran 15 in ihrer Position innerhalb der Verschlusskappe 16. Die Dichtmembran 15 wird somit innerhalb der Gassteuerungsvorrichtung 10 durch die aufgeklippte Verschlusskappe 16 auf dem Rand 24 des Umgehungskanals 21 angedrückt/gehalten. Die Dichtmembran 15 wird über die umlaufend ausgebildete, verstärkte Begrenzung 23 zwischen dem Rand 24 des Umgehungskanals 21 und der Verschlusskappe 16 eingeklemmt/ abgedichtet.

Bereiche der Dichtmembran 15, die sich von der Begrenzung 23 unterscheiden, sind berührungsfrei zu der Verschlusskappe 16 ausgebildet. Diese Bereiche, können auch als Beaufschlagungsbereiche bezeichnet werden, da die Schalteinrichtung diese Bereiche der Dichtmembran 15 mit dem über den zweiten Gaskanal zugeführten beaufschlagten Arbeitsdruck beaufschlagt.

Die Dichtmembran 15 wird in einem Ruhezustand (ohne Gasströme) durch das Gewicht und ihre Struktur auf der Öffnung 14 gehalten. In einem Störungsfall oder einer Exspiration ist die Schalteinrichtung ist eingerichtet, den zweiten Gaskanal 12 mit einem Arbeitsdruck zu beaufschlagungen.

Ist die Schalteinrichtung hingegen eingerichtet, den zweiten Gaskanal 12 beaufschlagungsfrei zu halten/zu schalten, werden die Beaufschlagungsbereiche der Dichtmembran 15 nicht mit einem Gasstrom beaufschlagt, sodass der Gasstrom des ersten Gaskanals 11 ausreichend ist, um die Dichtmembran im Bereich der Beaufschlagungsbereiche anzuheben und den Gasstrom in den Umgehungskanal 21 zu führen.

Ferner sind in der Figur 2 das Rückschlagventil 19 sowie der Umgehungskanal 21 dargestellt.

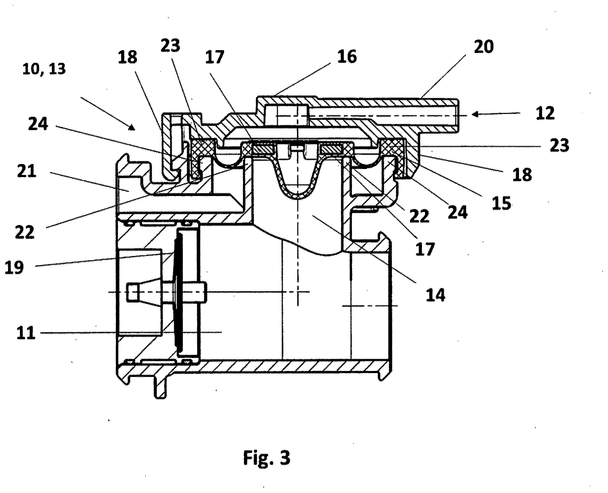

Figur 3 zeigt einen Längsschnitt durch die in den Figuren 1 und 2 gezeigte Schalteinrichtung 13. Dargestellt ist der erste Gaskanal 11 mit dem Rückschlagventil 19, der Öffnung 14 und der Dichtmembran 15 sowie der zweite Gaskanal 12. Zudem sind die Verschlusskappe 16 mit dem Anschluss 20 und der Umgehungskanal 21 dargestellt.

Über den ersten Gaskanal 11 kann ein inspiratorischer Gasstrom zu dem Patienten gefördert werden. Das Rückschlagventil 19 verhindert ein Rückströmen von Ausatemgas in das Beatmungsgerät über den ersten Gaskanal 11. Bei einer Blockade/Störung des exspiratorischen Zweiges des Beatmungsgerätes kann somit über den ersten Gaskanal 11 keine Exspiration des Gasstroms/Ausatemgases erfolgen.

Gezeigt ist zudem der Umgehungskanal 21, der sich von der Öffnung 14 aus erstreckt. Der Umgehungskanal 21 ist als umlaufender Kanal um die Öffnung 14 ausgebildet und erstreckt sich in seinem weiteren Verlauf zu einem Kanal, der sich in einer Richtung, parallel zu dem ersten Gaskanal 11 erstreckt. Durch die umlaufende Anordnung des Umgehungskanals kann eine große Menge an Ausatmengas gleichzeitig um das Rückschlagventil 19 vorbei und über die Öffnung 14 abgeführt werden. Der Umgehungskanal 21 kann in den inspiratorischen . Zweig einmünden oder als separater Zweig ausgebildet sein.

Die Dichtmembran 15 ist kreisförmig ausgebildet und weist in ihrem Zentrum ein Gewicht 17 auf. Das Gewicht 17 ist ringförmig/unterlegscheibenförmig ausgebildet. Das Gewicht 17 weist in seiner formbedingten Aussparung eine kegelförmige Ausgestaltung der Dichtmembran 15 auf, die sich in Richtung des ersten Gaskanals 11 erstreckt. Die kegelförmige Ausgestaltung bietet den Vorteil, dass die Dichtmembran 15 in ihrer Position auf der Öffnung 14 gegen verrutschen gesichert ist. Zudem bietet sie dem Patienten eine Einbauhilfe bezüglich der Orientierung der Dichtmembran 15.

In weiteren Ausführungsformen kann die Ausgestaltung der Dichtmembran 15 eine andere geometrische Form aufweisen, die geeignet ist, die Dichtmembran 15 in ihrer Position zu halten. Das Gewicht 17 ist in die Dichtmembran 15 integriert ausgebildet. Die Dichtmembran 15 weist im Bereich des Gewichts 17 eine größere Materialstärke auf. Dies bietet zum einen den Vorteil, dass das Gewicht 17 in die Dichtmembran 15 integrierbar ist und zum anderen durch die höhere Materialstärke ein zusätzliches Gewicht 17 erzeugt wird, dass die Dichtmembran 15 in ihrer Position hält. Das Gewicht 17 kann auch auf die Dichtmembran 15 aufgebracht ausgebildet sein.

Die in der Figur 3 gezeigte Verschlusskappe 16 ist auf der Öffnung 14 angeordnet, wobei die Verschlusskappe 16 im Bereich der Begrenzung 23 die Dichtmembran 15 auf den Rand des Umgehungskanals 21 andrückt, während die Dichtmembran 15 in den Beaufschlagungsbereichen berührungsfrei zu der Verschlusskappe 16 angeordnet ist.

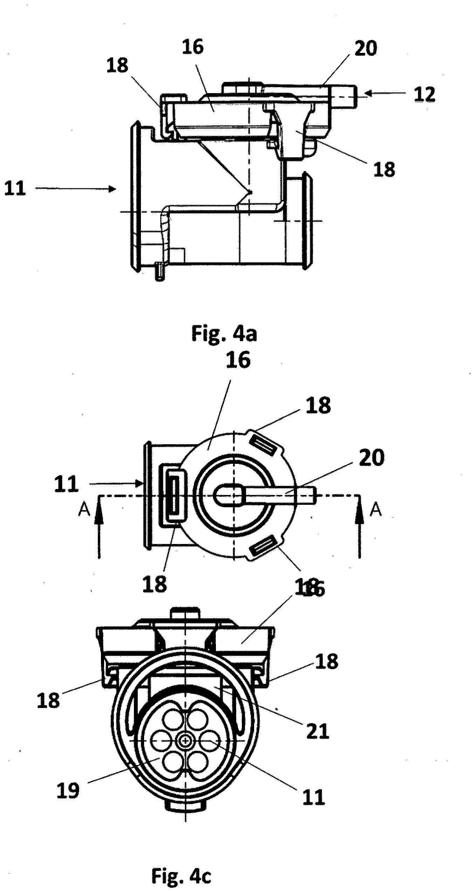

Figur 4a zeigt eine Seitenansicht der in den Figuren 1 bis 3 gezeigten Schalteinrichtung 13. Dargestellt ist der erste Gaskanal 11 sowie die Verschlusskappe 16 mit den Fortsätzen 18 und dem Anschluss 20 für den - nicht gezeigten - zweiten Gaskanal.

In der Figur 4a ist zu erkennen, dass die Eingangsseite des ersten Gaskanals 11 größer dimensioniert ist als die Ausgangsseite. Die Eingangsseite umfasst neben dem ersten Gaskanal 11 auch den zu einem Kanal verengten Umgehungskanal 21. Somit umfasst die Eingangsseite des ersten Gaskanals 11 sowohl den in Inspirationsflussrichtung ausgerichteten ersten Gaskanal 11 sowie den entgegen der Inspirationsflussrichtung ausgerichteten Umgehungskanal 21.

Figur 4b zeigt eine Draufsicht auf die erfindungsgemäße Verschlusskappe 16 der in den Figuren 1 bis 4b gezeigten erfindungsgemäßen Schalteinrichtung 13. Die Verschlusskappe 16 ist auf die - nicht gezeigte - Öffnung des ersten Gaskanals aufklippbar. Beim Aufklippen der Verschlusskappe 16 wird die Dichtmembran im Bereich des - nicht gezeigten - Gewichts von der Verschlusskappe 16 auf eine - in Figur 3 gezeigte - Erhebung 22 der Öffnung gedrückt und abgedichtet.

Die Verschlusskappe 16 weist einen Anschluss 20 für den - nicht gezeigten - zweiten Gaskanal 12 auf. Über den Anschluss 20 kann der Gasstrom des - nicht gezeigten - zweiten Gaskanals 12 auf die - nicht gezeigte - Dichtmembran aufgebracht werden, um die - nicht gezeigte - Öffnung zu verschließen. Die Verschlusskappe 16 umfasst ferner die Fortsätze 18 die zumeist Widerhaken ausbilden und eingerichtet sind in Ausnehmungen im Bereich der Öffnung einzugreifen. In weiteren Ausführungsformen kann die Verschlusskappe 16 weitere Fortsätze 18 aufweisen oder über ähnliche Verrastungselemente auf der Öffnung befestigbar sein.

Figur 4c zeigt eine Draufsicht auf das Rückschlagventil 19 der in den Figuren 1 bis 4b gezeigten erfindungsgemäßen Gassteuerungsvorrichtung 10. Dabei wird aus Richtung des einströmenden Gasstroms, in Inspirationsflussrichtung, auf das Rückschlagventil 19 geblickt. Gezeigt ist auch der Umgehungskanal 21, über welchen bei einer Blockade/Störung des exspiratorischen Zweiges des Beatmungsgerätes der Gasstrom um das Rückschlagventil 19 herumführbar ist.

Dargestellt ist auch die Verschlusskappe 16 in Seitenansicht mit den Fortsätzen 18, mittels derer die Verschlusskappe 16 auf die - nicht gezeigte - Öffnung aufklippbar ist.

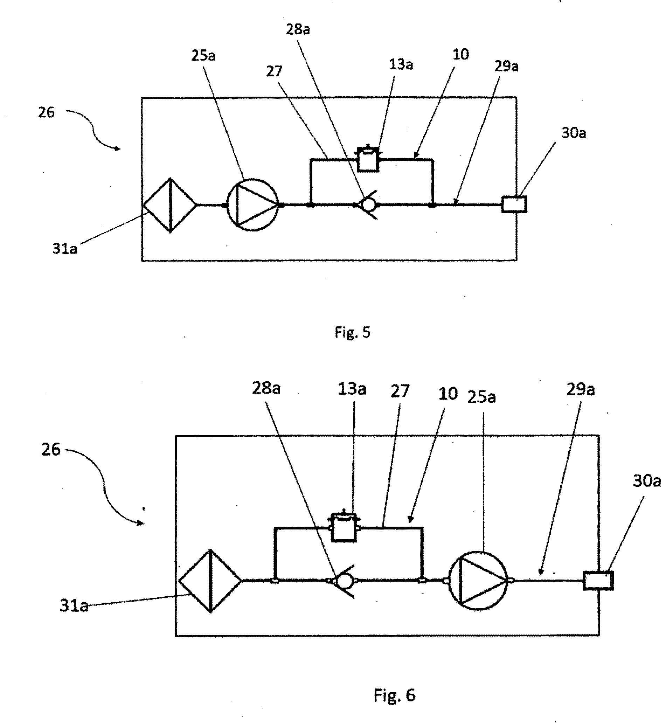

In den Figuren 5 bis 13 sind verschiedene Ausführungsformen pneumatischer Schaltanordnungen eines erfindungsgemäßen Beatmungsgerätes als Teil einer erfindungsgemäßen Gassteuerungsvorrichtung gezeigt.

Figur 5 zeigt eine Ausführungsform eines schematischen Aufbaus einer pneumatischen Schaltanordnung eines erfindungsgemäßen Beatmungsgerätes 26 umfassend eine erfindungsgemäße Gassteuerungsvorrichtung 10. Die pneumatische Anordnung des Beatmungsgerätes 26 umfasst einen inspiratorischen Atemgasweg 29a mit einem Bypass 27 und einem Rückschlagventil 28a. Derartige pneumatische Anordnungen werden in der Regel in einem Leckageschlauchsystem verwendet. Der inspiratorische Atemgasweg 29a umfasst einen Geräteeingang 31a und einen Geräteausgang 30a. Der Geräteausgang 30a ist dabei patientennah angeordnet und der Geräteeingang 31a ist patientenfern angeordnet. Von dem Geräteeingang 31a erstreckt sich zu dem Geräteausgang 30a der Atemgasweg 29a. Der Atemgasweg 29a umfasst einen Atemgasantrieb 25a und die Gassteuerungsvorrichtung 10 mit dem Rückschlagventil 28a und der Schalteinrichtung/dem Schaltventil 13a. Die Schalteinrichtung 13 ist in der erfindungsgemäßen Gassteuerungsvorrichtung 10 in einem Bypass 27 zu dem Rückschlagventil 28a angeordnet. Der Bypass 27 zweigt zwischen dem Atemgasantrieb 25a und dem Rückschlagventil 28a ab und mündet zwischen dem Rückschlagventil 28a und dem Geräteausgang 30a wieder in den inspiratorischen Atemgasweg 29a. Der Atemgasantrieb 25a ist zwischen dem Geräteeingang 31a und dem Rückschlagventil 28a angeordnet. Über die im Bypass 27 angeordnete Schalteinrichtung 13a kann Atemgas in Richtung des Geräteeingangs 31a rückgeführt werden, wodurch das Rückschlagventil 28a umgangen wird.

Figur 6 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Eine derartige Anordnung wird in der Regel in einem Leckageschlauchsystem verwendet. Gezeigt ist das erfindungsgemäße Beatmungsgerät 26 mit dem inspiratorischen Atemgasweg 29a mit dem Bypass 27 um dem Rückschlagventil 28a. Der inspiratorische Atemgasweg 29a umfasst den Geräteeingang 31a und den Geräteausgang 30a. Von dem Geräteeingang 31a erstreckt sich zu dem Geräteausgang 30a der Atemgasweg 29a. Der Atemgasweg 29a umfasst das den Atemgasantrieb 25a und die Gassteuerungsvorrichtung 10 mit dem Rückschlagventil 28a und der Schalteinrichtung/dem Schaltventil 13a. Die Schalteinrichtung 13a ist in dem Bypass 27 zu dem Rückschlagventil 28a angeordnet. Der Bypass 27 zweigt in dieser alternativen Anordnung zwischen dem Geräteeingang 31a und dem Rückschlagventil 28a ab und mündet zwischen dem Rückschlagventil 28a und dem Atemgasantrieb 25a wieder in den inspiratorischen Atemgasweg 29a. Der Atemgasantrieb 25a ist zwischen dem Rückschlagventil 28a und dem Geräteausgang 30a angeordnet. Über die im Bypass 27 angeordnete Schalteinrichtung 13a kann Atemgas in Richtung des Geräteeingangs 31a rückgeführt werden, nachdem es den Atemgasantrieb 25a passiert hat.

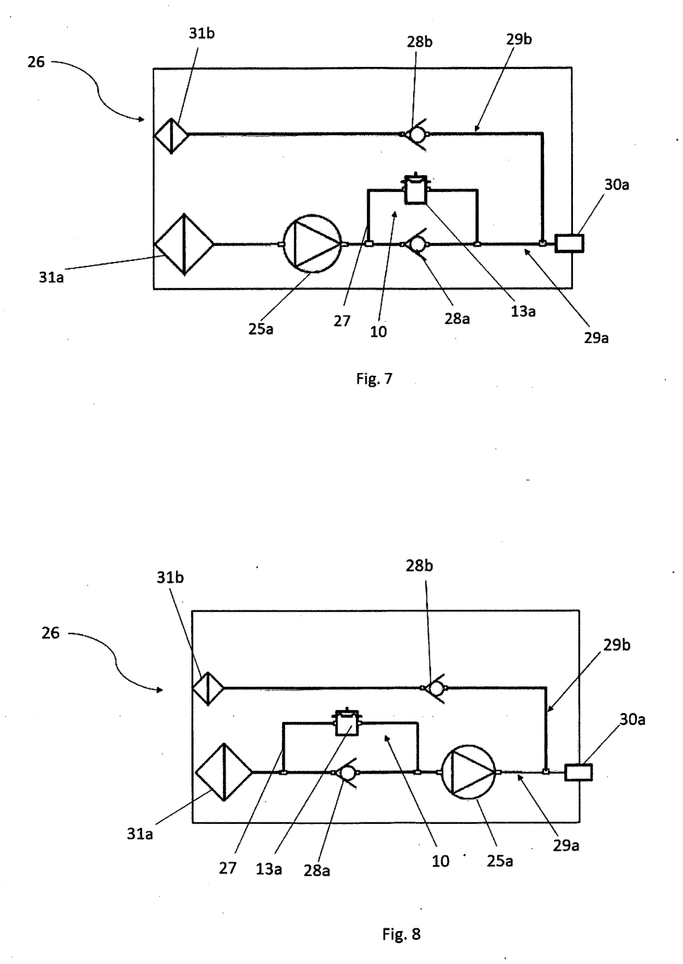

Figur 7 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Eine derarti 29bge Anordnung kann in einem Ventilschlauchsystem verwendet werden. Gezeigt ist das erfindungsgemäße Beatmungsgerät 26, mit dem inspiratorischen Atemgasweg 29a, dem Bypass 27 und einem zweiten inspiratorischen Atemgasweg 29b. Der inspiratorische Atemgasweg 29a erstreckt sich von dem Geräteeingang 31a zu dem Geräteausgang 30a. Der inspiratorische Atemgasweg 29a umfasst den Atemgasantrieb 25a und die Gassteuerungsvorrichtung 10 mit dem Rückschlagventil 28a und der Schalteinrichtung 13a, wobei die Schalteinrichtung 13a in dem Bypass 27 zur Umgehung des Rückschlagventils 28a angeordnet ist. Die in Figur 7 gezeigte Ausführungsform des Beatmungsgerätes 10 weist den zweiten inspiratorischen Atemgasweg 29b auf, der sich von einem zweiten Geräteeingang 31b zu dem gemeinsamen Geräteausgang 30a erstreckt. Der zweite inspiratorische Atemgasweg 29b mündet zwischen dem Rückschlagventil 28a und dem gemeinsamen Geräteausgang 30a in den inspiratorischen Atemgasweg 28a ein. Der zweite inspiratorische Atemgasweg 29b kann ein Rückschlagventil 28b umfassen. Über den zweiten inspiratorischen Atemgasweg 29b kann beispielsweise bei einer Blockade/Störung des inspiratorischen Atemgasweges 29a Atemgas über den separaten Geräteeingang 31b bezogen werden und dem Patienten zugeführt werden. Zusätzlich kann bei einer Blockade eines exspiratorischen Atemgasweges Atemgas durch Umgehung des Rückschlagventils 28a durch die Schalteinrichung 13a rückgeführt werden und dem Patienten eine Ausatmung ermöglicht werden.

Figur 8 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Gezeigt ist das erfindungsgemäße Beatmungsgerätes 26, mit dem inspiratorischen Atemgasweg 29a, dem Bypass 27 und dem zweiten inspiratorischen Atemgasweg 29b. Bei dieser alternativen Ausführungsform ist in dem inspiratorischen Atemgasweg 29a das Rückschlagventil 28a zwischen dem Geräteeingang 31a und dem Atemgasantrieb 25a angeordnet. Um das Rückschlagventil 28a ist der Bypass 27 ausgebildet, der zwischen dem Geräteeingang 31a abzweigt und zwischen dem Rückschlagventil 28a und dem Atemgasantrieb 25a wieder in den inspiratorischen Atemgasweg 29a einmündet. Der Atemgasantrieb 25a ist zwischen dem Rückschlagventil 28a und dem Geräteausgang 30a angeordnet. Der zweite inspiratorische Atemgasweg 29b mündet zwischen dem Atemgasantrieb 25a und dem gemeinsamen Geräteausgang 30a in den inspiratorischen Atemgasweg 29a ein. Der zweite inspiratorische Atemgasweg 29b kann ein Rückschlagventil 28b umfassen. Über den zweiten inspiratorischen Atemgasweg 28b kann auch bei dieser Ausführungsform beispielsweise bei einer Blockade/Störung des inspiratorischen Atemgasweges 29a Atemgas über den separaten Geräteeingang 31b bezogen werden und dem Patienten zugeführt werden. Zusätzlich kann ebenfalls bei einer Blockade eines exspiratorischen Atemgasweges Atemgas durch Umgehung des Rückschlagventils 28a durch die Gassteuerungsvorrichtung mit der Schalteinrichtung 13a rückgeführt werden und dem Patienten eine Ausatmung ermöglicht werden.

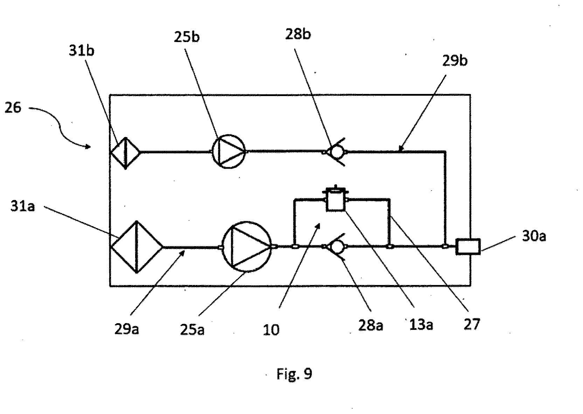

Figur 9 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10.Gezeigt ist das erfindungsgemäße Beatmungsgerät 26, mit dem inspiratorischen Atemgasweg 29a, dem Bypass 27 und dem zweiten inspiratorischen Atemgasweg 29b.

Der inspiratorische Atemgasweg 29a erstreckt sich von dem Geräteeingang 31a zu dem Geräteausgang 30a und umfasst den Atemgasantrieb 25a, und die erfindungsgemäße Gassteuerungsvorrichtung 10 mit dem Rückschlagventil 28a und der Schalteinrichtung 13a.

Das Schaltventil 28a ist in dem Bypass 27 zur Umgehung des Rückschlagventils 28a angeordnet. Der Bypass 27 ist zwischen dem Atemgasantrieb 25a und dem Geräteausgang 30a ausgebildet. Die in Figur 9 gezeigte Ausführungsform des Beatmungsgerätes 26 weist ebenfalls den zweiten inspiratorischen Atemgasweg 29b auf, der sich von dem zweiten Geräteeingang 31b zu dem gemeinsamen Geräteausgang 30a erstreckt. Der zweite inspiratorische Atemgasweg 29b mündet zwischen dem Rückschlagventil 28b und dem gemeinsamen Geräteausgang 30a in den inspiratorischen Atemgasweg 29a ein. Der zweite inspiratorische Atemgasweg 29b weist ein Rückschlagventil 28b und einen Atemgasantrieb 25b auf, wobei der Atemgasantrieb 25b zwischen dem zweiten Geräteeingang 31b und dem Rückschlagventil 28b angeordnet ist. Über den zweiten inspiratorischen Atemgasweg 29b kann auch bei dieser Ausführungsform beispielsweise bei einer Blockade/Störung des inspiratorischen Atemgasweges 29a Atemgas über den separaten Geräteeingang 31b bezogen werden und dem Patienten zugeführt werden. Zusätzlich kann ebenfalls bei einer Blockade eines exspiratorischen Atemgasweges Atemgas durch Umgehung des Rückschlagventils 28a über das im Bypass 27 angeordnete Schaltventil 13a rückgeführt werden und dem Patienten eine Ausatmung ermöglicht werden.

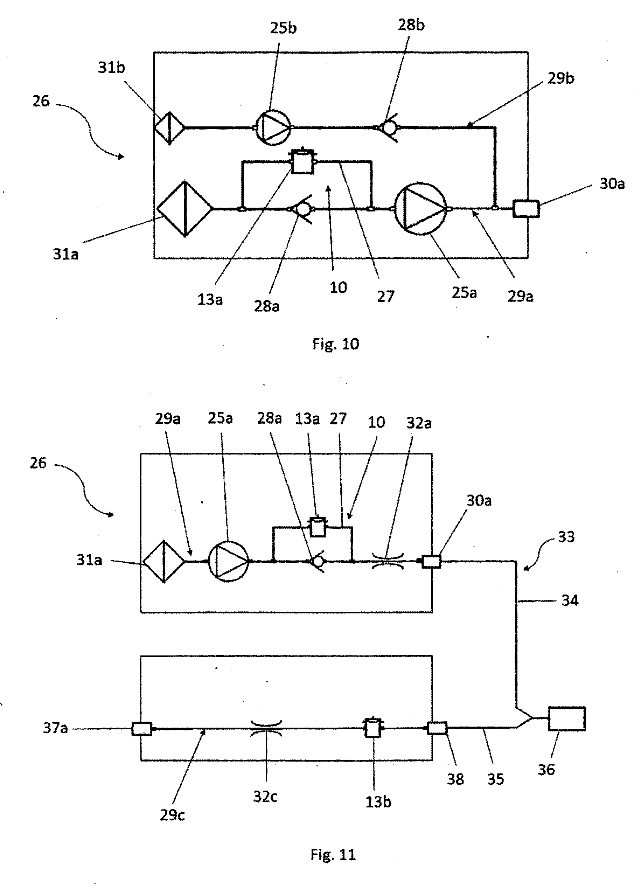

Figur 10 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Gezeigt ist ein erfindungsgemäßes Beatmungsgerät 26, mit dem inspiratorischen Atemgasweg 29a, dem Bypass 27 und dem zweiten inspiratorischen Atemgasweg 29b. Der inspiratorische Atemgasweg 29a erstreckt sich von dem Geräteeingang 31a zu dem Geräteausgang 30a und umfasst die Gassteuerungsvorrichtung 10 mit dem Rückschlagventil 28a und der Schalteinrichtung 13a sowie den Atemgasantrieb 25a.

Die Schalteinrichtung 13a ist in dem Bypass 27 zur Umgehung des Rückschlagventils 28b angeordnet. Der Bypass 27 mit der Schalteinrichtung 13a ist zwischen dem Geräteingang 31a und dem Atemgasantrieb 25a angeordnet. Die in Figur 10 gezeigte Ausführungsform des Beatmungsgerätes 26 weist den zweiten inspiratorischen Atemgasweg 29b auf, der sich von einem zweiten Geräteeingang 31b zu dem gemeinsamen Geräteausgang 30a erstreckt. Der zweite inspiratorische Atemgasweg 29b mündet zwischen dem Atemgasantrieb 25a und dem gemeinsamen Geräteausgang 30a in den inspiratorischen Atemgasweg 29a ein. Der zweite inspiratorische Atemgasweg 29b weist das Rückschlagventil 28b und den Atemgasantrieb 25b auf, wobei der Atemgasantrieb 25b zwischen dem zweiten Geräteeingang 31b und dem Rückschlagventil 28b angeordnet ist.

Figur 11 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Gezeigt ist das erfindungsgemäße Beatmungsgerät 26 mit dem inspiratorischen Atemgasweg 29a mit einem Bypass 27 und einem exspiratorischen Atemgasweg 29c.

Der inspiratorische Atemgasweg 29a erstreckt sich von dem Geräteeingang 31a hin zu dem Geräteausgang 30a. Der inspiratorische Atemgasweg 29a umfasst dabei den Atemgasantrieb 25a sowie die Gassteuerungsvorrichtung 10 mit dem Rückschlagventil 28a und der Schalteinrichtung 13a, welche in einem Bypass 27 zu dem Rückschlagventil 28a angeordnet ist sowie eine Gasmesseinrichtung (Fluss und/oder) Druck 32a. Die Gasmesseinrichtung 32a ist zwischen dem Bypass 27 und dem Geräteausgang 30a angeordnet. Über die Gasmesseinrichtung 32a kann ein Fluss bzw. ein Volumen oder ein Druck des Atemgases im Atemgasweg erfasst werden. Bei einem vorgegebenen Volumen oder Druck an Atemgas über einen vorgegebenen Zeitraum ist die Schalteinrichtung 13a beispielsweise mittels eines 3/2-Wegeventils schaltbar. Die Schalteinrichtung 13a ist in der Regel eingerichtet und ausgebildet einen PEEP (positive end-expiratory pressure) im Bereich von 0-20hPa, bevorzugt 0-15hPa einzustellen.

Der exspiratorische Atemgasweg 29c erstreckt sich von einem exspiratorischen Geräteeingang 38 zu dem exspiratorischen Geräteausgang 37a und umfasst eine Schalteinrichtug 13b und eine Flussmesseinrichtung 32c. Die Flussmesseinrichtung 32c ist dabei zwischen dem exspiratorischen Geräteausgang 37a und der Schalteinrichtung 13b angeordnet. Die Flussmesseinrichtung 32c dient dabei der Erfassung eines Flusses bzw. eines Volumens oder eines Druckes des Atemgases im Atemgasweg. Basierend auf den durch die Flussmesseinrichtung 32c erfassten Werte kann eine Rückmeldung über das Volumen des durch den Patienten abgegebenen Atemgases an das Beatmungsgerät erfolgen.

An den Geräteausgang 30a ist ein Schlauchsystem 33 mit einem ersten Zweig 34 und einem zweiten Zweig 35 adaptiert. Der erste Zweig 34 führt von dem Geräteausgang 30a zu einem Patienteninterface 36. Vor dem Patienteninterface 36 führt das Schlauchsystem 33 mit dem zweiten Zweig 35 zu dem exspiratorischen Geräteeingang 38 für exspiratorisches Atemgas. Über das Schlauchsystem 33 ist das Patienteninterface 36 mit dem inspiratorischen Atemgasweg 29a und dem exspiratorischen Atemgasweg 29c verbunden.

Bei der in Figur 11 gezeigten Ausführungsform des Beatmungsgerätes 26 kann ein Atemgas über den Geräteeingang 31a bezogen werden und über den Atemgasantrieb 25a über das Rückschlagventil 28a in Richtung des Geräteausgangs 30a geführt werden.

Das Atemgas wird von dem Geräteausgang 30a über den Zweig 34 des adaptierten Schlauchsystems 33 zu dem Patienteninterface 36 weitergeleitet, worüber ein Patient das Atemgas einatmen kann. Die ausgeatmete Gas des Patienten kann über den Zweig 35 des adaptierten Schlauchsystems 33 über den exspiratorischen Geräteeingang 38 dem Atemgasweg 29c zugeführt werden. Über den exspiratorischen Atemgasweg 29c wird das Atemgas hin zu dem exspiratorischen Geräteausgang 37a geführt und an die Umgebung abgegeben. Bei einer Blockade/Störung des exspiratorischen Atemgasweges 29c kann das Atemgas durch Steuerung der Schalteinrichtung 13a in dem Bypass 27 in dem inspiratorischen Atemgasweg 29a abgeführt werden.

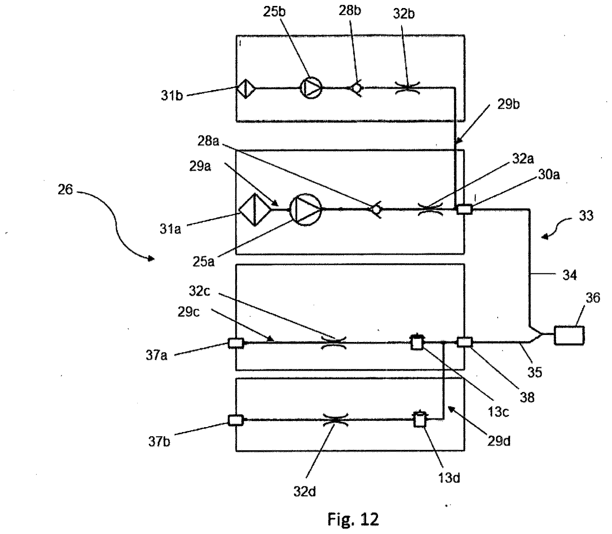

Figur 12 zeigt eine weitere Ausführungsform eines schematischen Aufbaus einer pneumatischen Schaltanordnung des erfindungsgemäßen Beatmungsgerätes 26 umfassend die erfindungsgemäße Gassteuerungsvorrichtung 10. Gezeigt ist ein erfindungsgemäßes Beatmungsgerät 26 mit dem ersten inspiratorischen Atemgasweg 29a und dem zweiten inspiratorischen Atemgasweg 29b sowie mit dem exspiratorischen Atemgasweg 29c und einem separaten exspiratorischen Atemgasweg 29d.

Der inspiratorische Atemgasweg 29a erstreckt sich von dem Geräteeingang 31a hin zu dem Geräteausgang 30a. Der inspiratorische Atemgasweg 29a umfasst den Atemgasantrieb 25a, das Rückschlagventil 28a sowie eine Flussmesseinrichtung 32a. Die Flussmesseinrichtung 32a ist zwischen dem Rückschlagventil 28a und dem Geräteausgang 30a angeordnet. Die Flussmesseinrichtung 32a erfasst einen Fluss bzw. ein Volumen oder einen Druck des Atemgases welches über den inspiratorischen Atemgasweg 29a an den Patienten abgegeben wird.

Die in Figur 12 gezeigte Ausführungsform des Beatmungsgerätes 26 weist einen zweiten inspiratorischen Atemgasweg 29b auf, der sich von dem zweiten Geräteeingang 31b zu dem gemeinsamen Geräteausgang 30a erstreckt. Der zweite inspiratorische Atemgasweg 29b mündet zwischen der Flussmesseinrichtung 32a und dem gemeinsamen Geräteausgang 30a in den inspiratorischen Atemgasweg 29ä ein. Der zweite inspiratorische Atemgasweg 29b kann den separaten Atemgasantrieb 25b, ein Rückschlagventil 28b und eine Flussmesseinrichtung 32b umfassen. Der separate Atemgasantrieb 25b ist zwischen dem zweiten Geräteeingang 31b und dem Rückschlagventil 28b angeordnet. Die Flussmesseinrichtung 32b kann ein Volumen oder Druck des inspiratorischen Atemgases in dem Atemgasweg 29b an erfassen und an das Beatmungsgerät 26 übermitteln. Über den zweiten inspiratorischen Atemgasweg 29b kann bei einer Blockade des inspiratorischen Atemgasweges 29a Atemgas über den Geräteeingang 31b bezogen und dem Patienten zugeführt werden.

Die in Figur 12 gezeigte Ausführungsform des Beatmungsgerätes 26 weist ferner den exspiratorischen Atemgasweg 29c auf, der sich von dem exspiratorischen Geräteeingang 38 zu dem exspiratorischen Geräteausgang 37a erstreckt und die Schalteinrichtung 13b und die Flussmesseinrichtung 32c umfasst. Die Flussmesseinrichtung 32c ist dabei zwischen dem exspiratorischen Geräteausgang 37a und der Schalteinrichtung 13b angeordnet. Die Flussmesseinrichtung 32c erfasst ein Volumen und/oder einen Druck des Atemgases, welches der Patient abgibt. Über den exspiratorischen Atemgasweg 29c ist ein von dem Patienten abgegebenes Atemgas abführbar.

Die Gasmesseinrichtungen 32a, 32b, 32c und 32d können eingerichtet sein, einen Fluss bzw. ein Volumen oder einen Druck des Atemgases in dem Atemgasweg zu erfassen und basierend auf diesem ein Schaltventil zu schalten bzw. zu öffnen und zu schließen oder eine Rückmeldung an das Beatmungsgerät 26 über den Fluss bzw. das Volumen oder den Druck des dem Patienten zugeführten Atemgases oder von dem Patienten abgegebenes Atemgases zu liefern.

Die in Figur 12 gezeigte Ausführungsform der pneumatischen Anordnung in dem Beatmungsgerät 26 weist zudem einen separaten exspiratorischen Atemgasweg 29d auf. Der separate exspiratorische Atemgasweg 29d zweigt von dem exspiratorischen Atemgasweg 29c ab und erstreckt sich hin zu einem separaten Geräteausgang 37b. Der separate exspiratorische Atemgasweg 29d umfasst eine Schalteinrichtung 14c und eine Flussmesseinrichtung 32d. Die Messeinrichtung 32d erfasst einen Fluss bzw. ein Volumen oder einen Druck des Atemgases, welches der Patient abgibt. Über den separaten exspiratorischen Atemgasweg 16d ist bei einer Blockade/Störung des exspiratorischen Atemgaswegs 29c von dem Patienten abgegebenes Atemgas abführbar.

An den Geräteausgang 30a ist ein Schlauchsystem 33 mit einem ersten Zweig 34 und einem zweiten Zweig 35 adaptiert. Der erste Zweig 34 führt von dem Geräteausgang 30a zu einem Patienteninterface 36. Vor dem Patienteninterface 36 führt das Schlauchsystem 33 mit dem zweiten Zweig 35 zu dem exspiratorischen Geräteeingang 38 für exspiratorisches Atemgas. Über das Schlauchsystem 33 ist das Patienteninterface 36 mit dem inspiratorischen Atemgasweg 29a, dem zweiten inspiratorischen Atemgasweg 29b, dem exspiratorischen Atemgasweg 29c und dem separaten exspiratorischen Atemgasweg 29d verbunden.

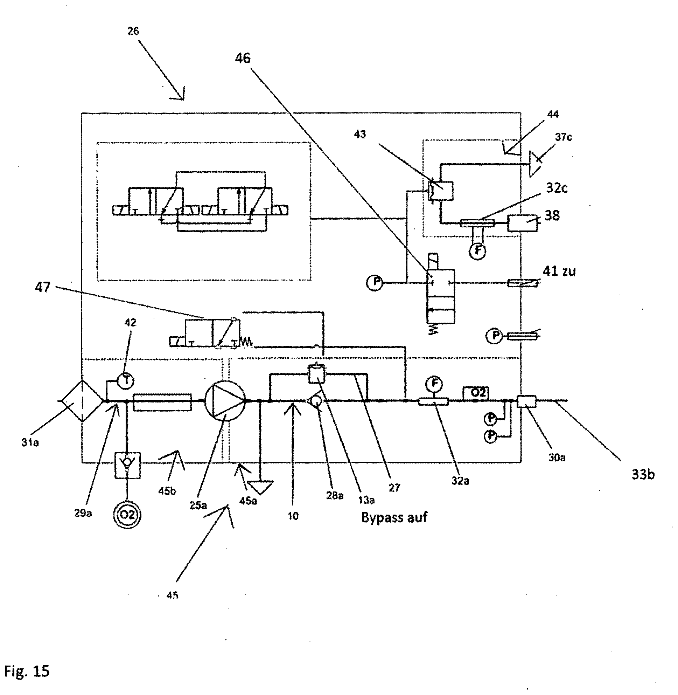

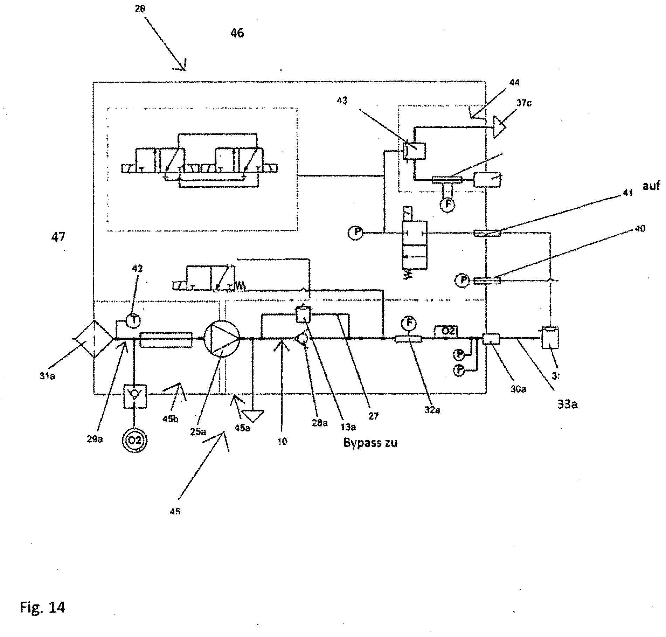

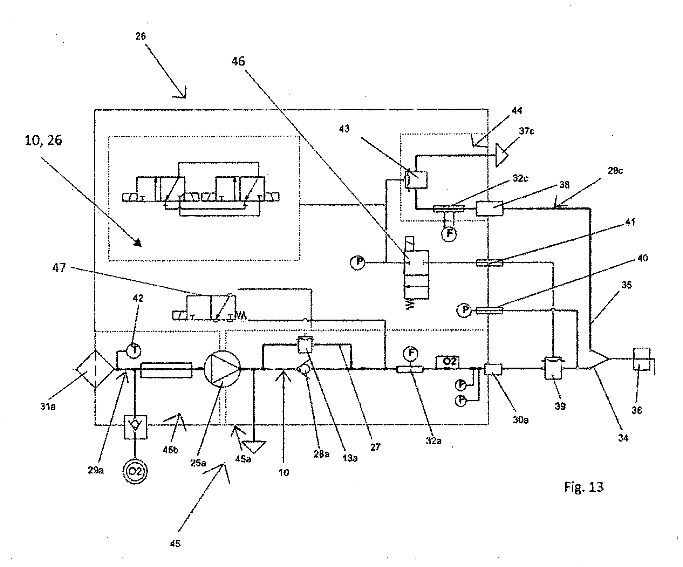

Figuren 13 - 15 zeigt eine weitere Ausführungsform eines schematischen Aufbaus des erfindungsgemäßen Beatmungsgerätes 26 umfassend die erfindungsgemäße Gassteuerungsvorrichtung 10.

Gezeigt ist eine pneumatische Schaltanordnung umfassend den inspiratorischen Atemgasweg 29b und den exspiratorischen Atemgasweg 29c.

Das Beatmungsgerät umfasst einen Atemgasweg 29a der sich zwischen einem Geräteeingang 31a und einem Geräteausgang 30a erstreckt, wobei der Atemgasweg 29a einen Atemgasantrieb 25a und eine Gassteuerungsvorrichtung 10 mit einem Rückschlagventil 28a, einem Bypass 27 und einer Schalteinrichtung 13a umfasst, wobei der Atemgasantrieb 25a eine inspiratorische Atemgasströmung von dem Geräteeingang 31a durch das Rückschlagventil zu dem Geräteausgang 30a führt, wobei das Rückschlagventil eingerichtet und angeordnet ist eine Atemgasströmung (in Sperrrichtung des Rückschlagventils) in Richtung des Geräteeingangs 31a zu verhindern und der Bypass 27 eingerichtet und angeordnet ist eine Atemgasströmung zur Umgehung des Rückschlagventils 28a zu ermöglichen und das Schalteinrichtung 13a eingerichtet und angeordnet ist eine Atemgasströmung durch den Bypass 27 zur Umgehung des Rückschlagventils 28a zumindest zeitweise zu ermöglichen oder zu unterbrechen.

Die Atemgasströmung verläuft durch den Bypass entgegen der Sperrrichtung des Rückschlagventils.

Die Schalteinrichtung 13a ist in dem Bypass 27 angeordnet und der Bypass 27 zweigt von dem Atemgasweg 29a, vor dem Rückschlagventil 28a ab und mündet hinter dem Rückschlagventil 28a wieder in den Atemgasweg 29a oder in den Geräteeingang.

Der Atemgasantrieb 25a ist zwischen dem Geräteeingang 31a und dem Rückschlagventil 28a angeordnet.

Der Atemgasantrieb 25a kann auch zwischen Rückschlagventil 28a und dem Geräteausgang 30a angeordnet sein.

Die Schalteinrichtung 13 ist in dem Bypass 27 angeordnet und der Bypass 27 zweigt beispielsweise zwischen dem Atemgasantrieb 25a und dem Rückschlagventil 28a ab und mündet zwischen dem Rückschlagventil 28a und dem Geräteausgang 30a wieder in den Atemgasweg 29a, wobei der Atemgasantrieb 25a zwischen dem Geräteeingang 31a und dem Rückschlagventil 28a angeordnet ist und über die im Bypass 27 angeordnete Schalteinrichtung 13a Atemgas in Richtung des Geräteeingangs 31a rückgeführt werden kann, wodurch das Rückschlagventil 28a umgangen wird.

Denkbar ist auch ein Aufbau, bei dem die Schalteinrichtung 13a in dem Bypass 27 angeordnet ist und der Bypass 27 zwischen dem Geräteeingang 31a und dem Rückschlagventil 28a abzweigt und zwischen dem Rückschlagventil 28a und dem Atemgasantrieb 25a wieder in den Atemgasweg 29a mündet, wobei der Atemgasantrieb 25a zwischen dem Rückschlagventil 28a und dem Geräteausgang 30a angeordnet ist und über die im Bypass 27 angeordnete Schalteinrichtung 13a Atemgas in Richtung des Geräteeingangs 31a rückgeführt werden kann, nachdem es den Atemgasantrieb 25a passiert hat.

Denkbar ist auch, dass ein zweiter Atemgasweg 29b von einem Geräteeingang 31a, 31b zu dem Geräteausgang führt 30a, wobei der zweite Atemgasweg 29b vor dem Geräteausgang und stromabwärts hinter dem Rückschlagventil und hinter dem Atemgasantrieb in den ersten Atemgasweg 29a mündet.

Denkbar ist auch, dass in dem zweiten Atemgasweg 29b ein Rückschlagventil 28b und/oder ein Atemgasantrieb 25b angeordnet ist, um einen definierten Atemgasstrom von dem Geräteeingang 31a, 31b zu dem Geräteausgang 30a zu führen.

Denkbar ist auch, dass eine Gasmesseinrichtung 32a zwischen dem Bypass 27 und dem Geräteausgang 30a angeordnet ist und eingerichtet ist einen Fluss oder ein Volumen oder einen Druck des Atemgases im Atemgasweg zu erfassen und die Schalteinrichtung 13a eingerichtet und ausgebildet ist einen PEEP (positive end-expiratory pressure) im Bereich von 0-20hPa, bevorzugt 0-15hPa einzustellen.

Denkbar ist auch, dass das Beatmungsgerät einen exspiratorischen Atemgasweg 29c aufweist, der sich von einem exspiratorischen Geräteeingang 38 zu einem exspiratorischen Geräteausgang 37a erstreckt und eine Schalteinrichtung 13b und eine Gasmesseinrichtung 32c umfasst, wobei die Gasmesseinrichtung 32c zwischen dem exspiratorischen Geräteausgang 37a und der Schalteinrichtung 13b angeordnet ist.

Denkbar ist auch, dass ein Schlauchsystem 33 an den Geräteausgang 30a montiert ist, wobei das Schlauchsystem 33 einen ersten Zweig 34 und einem zweiten Zweig 35 aufweist, wobei der erste Zweig 34 von dem Geräteausgang 30a zu einem Patienteninterface 36 führt und von dem Patienteninterface 36 ein zweiter Zweig 35 zu dem exspiratorischen Geräteeingang 38 führt, wobei über den ersten Zweig 34 inspiratorisches Atemgas aus dem inspiratorischen Atemgasweg 29a zu dem Patienteninterface 36 geführt wird und über den zweiten Zweig 35 exspiratorisches Atemgas aus dem Patienteninterface 36 zu dem exspiratorischen Atemgasweg 29a geführt wird.

Denkbar ist auch, dass bei einer Blockade/Störung des exspiratorischen Atemgasweges 29c das exspiratorische Atemgas durch Steuerung der Schalteinrichtung 13a in den inspiratorischen Atemgasweg 29a und über den geöffneten Bypass 27 in die Umgebung abgeführt werden kann.

Denkbar ist auch, dass das Beatmungsgerät einen ersten inspiratorischen Atemgasweg 29a und einen zweiten inspiratorischen Atemgasweg 29b sowie einen exspiratorischen Atemgasweg 29c und einen separaten exspiratorischen Atemgasweg 29d aufweist.

Der in Figur 13 gezeigte Aufbau einer Schaltanordnung ist eingerichtet, Atemgas von dem Geräteeingang über den inspiratorischen Atemgasweg 29a zu dem Geräteausgang 30a zu führen und über ein Schlauchsystem, welches auf den Geräteausgang gesteckt wird, dem Patienten zuzuführen. Ist das Schlauchsystem ein Zweischlauchsystem 33c wird ausgeatmetes Atemgas des Patienten über den Zweig 35 des Zweischlauchsystem 33c zu dem exspiratorischen Atemgasweg 29c gezuführt. Dazu ist der Zweig 35 des Zweischlauchsystems 33c an dem Eingang 38 des Beatmungsgeräts befestigt.

Der erste Zweig 34 des Zweischlauchsystem 33c ist am Geräteausgang befestigt und liefert inspiratorisches Atemgas zum Patienteninterface 36.

Der erste Zweig 34 kann mindestens einen separaten Zweig umfassen, der einen Drucksensor 40 und/oder einen Patientenventilanschluss umfasst. Beispielsweise kann der separate Zweig zwischen einem Schaltventil 39 und der Zusammenführung des ersten Zweiges 34 und des zweiten Zweiges 35 von dem ersten Zweig 34 abzweigen.

Der inspiratorische Atemgasweg 29a erstreckt sich von dem Geräteeingang 31a zu dem Geräteausgang 30a. Der inspiratorische Atemgasweg 29b umfasst dabei das Gebläse 25a und zumindest teilweise die Gassteuerungsvorrichtung 10 mit dem Rückschlagventil 28a und der Schalteinrichtung 13a. In der vorliegenden Ausführungsform umfasst der inspiratorische Atemgasweg 29a zudem einen Temperaturfühler 42, mindestens eine Flussmesseinrichtung 32a und/oder mindestens einen Drucksensor 40. Der inspiratorische Atemgasweg 29a kann in einem inspiratorischen Block 45 angeordnet sein, wobei der inspiratorische Block 45 eine Druckseite 45a und eine Saugseite 45b umfasst.

Der exspiratorische Atemgasweg 29c erstreckt sich ausgehend von dem zweiten Zweig 35, wenn das Zweischlauchsystem angeschlossen ist, in Richtung eines exspiratorischen Geräteausganges 37b. Dabei umfasst der exspiratorische Atemgasweg 29c einen exspiratorischen Geräteeingang 38, eine Flussmesseinrichtung 32c, ein internes Patientenventil 43 und einen exspiratorischen Geräteausgang 37c. In der vorliegenden Ausführungsform ist der exspiratorische Atemgasweg 29c in einem exspiratorischen Block 44 angeordnet. Optional kann der exspiratorische Block 44 getrennt von dem inspiratorischen Block 45 gesteuert werden oder aus dem Beatmungsgerät 26 entnehmbar sein.

Die Gassteuerungsvorrichtung 10 ist beispielsweise eingerichtet und ausgebildet den inspiratorischen Block 45 und den exspiratorischen Block zu steuern.

Das Beatmungsgerät ist zur Verwendung mit alternativen Schlauchsystemen 33 eingerichtet, wobei das Beatmungsgerät zumindest eine Atemgasquelle 25, zumindest einem Atemgasweg 27, 29, zumindest einen Geräteausgang 30, und einen Geräteeingang 31 umfasst und eine Gassteuervorrichtung 10 zur Voreinstellung des Beatmungsgerätes für die Verwendung der alternativen Schlauchsysteme 33 durch Schaltung von zumindest einer Schalteinrichtung 13, 46, 47, wobei für die Verwendung eines:
- Leckagesystems 33b die Gassteuerungsvorrichtung (10) die Schalteinrichtung 13a, 47 aktiviert den Atemgasweg 27 (Bypass) für einen Atemgasstrom zu öffnen
- Ventilsystems 33a mit einem Patientenventil 39 die Gassteuerungsvorrichtung (10) die Schalteinrichtung 13a, 47 aktiviert einen Atemgasstrom durch den Atemgasweg 27 zu sperren und die Schalteinrichtung 46 den Anschluss 41 öffnet, um einen Steuerdruck auf das Patientenventil 39 zu schalten
- Zweischlauchsystems die Gassteuerungsvorrichtung (10) die Schalteinrichtung 13a, 47 aktiviert einen Atemgasstrom durch den Atemgasweg 27 zu sperren und die Schalteinrichtung 46 den Anschluss 41 sperrt.

Die Gassteuervorrichtung 10 ist pneumatisch und/oder elektronisch ausgeführt und entsprechend einer Anwendervorgabe, über eine (nicht gezeigte) Anwenderschnittstelle, oder automatisch, wenn die Schlauchsysteme aufgrund technischer Kennungen von einem Erkennungssystem des Beatmungsgerätes erkannt werden, eine Voreinstellung des Beatmungsgerätes für die Verwendung der alternativen Schlauchsysteme 33 vornimmt.

Die Gassteuervorrichtung 10 umfasst zumindest das Rückschlagventil 28a, den Bypass und das Ventil 13a.

Die Gassteuervorrichtung 10 umfasst alle Ventile und Schalteinrichtungen und Atemgasquellen.

Der Atemgasweg 27, 29 und der Geräteausgang 30, und der Anschluss 41 und die zumindest eine Schalteinrichtung 46, 47 sind umbaufrei und adapterlos eingerichtet und ausgebildet, sodass ein Wechsel der Schlauchsysteme 33 ohne Adapter oder ohne Werkzeuge erfolgt. Die Schalteinrichtung 47 ist als Ventil ausgeführt, welches zumindest zeitweise den Rückatemdruck der Exspiration des Patienten als Steuerdruck auf das Ventil 13a schaltet. Die Schalteinrichtung 46 ist als Verschlussventil ausgeführt, welches den Steuerdruck, der aus der Atemgasquelle 25 kommt, auf den Druckstutzen 41 schaltet, wenn dieser mit dem Patientenventil 39 des Einschlauchsystems verbunden ist.

Der Drucksensor 40 wird bei Verwendung eines Leckagesystems 33b inaktiv geschaltet, kann jedoch auch aktiv bleiben, wenn das Schlauchsystem einen Druckmesspunkt aufweist.

Der Flusssensor 32c ist bei Verwendung eines Leckagesystems 33b inaktiv geschaltet oder wird nicht verwendet.

Der Flusssensor 32c wird bei Verwendung eines Einschlauchsystems 33a inaktiv geschaltet. Der Steuerdruck für die Ventile 13a, 39, und 43 kann zumindest teilweise oder zeitweise vom Gebläse 25a oder einem separaten Gebläse oder von der Rückatmung des Patienten entnommen werden.

Die Figuren 13 bis 15 zeigen:
Die Gassteuerungsvorrichtung 10 ist eingerichtet und ausgebildet für die Verwendung eines:
- Leckagesystems 33b die Schalteinrichtung 13a, 47 zu aktivieren den Atemgasweg 27 (Bypass) für einen Atemgasstrom zu öffnen
- Ventilsystems 33a mit einem Patientenventil 39 die Schalteinrichtung 13a, 47 zu aktivieren einen Atemgasstrom durch den Atemgasweg 27 zu sperren und die Schalteinrichtung 46 aktiviert den Anschluss 41 zu öffnen, um einen Steuerdruck auf das Patientenventil 39 zu schalten
- Zweischlauchsystems die Schalteinrichtung 13a, 47 zu aktivieren einen Atemgasstrom durch den Atemgasweg 27 zu sperren und die Schalteinrichtung 46 zu aktivieren den Anschluss 41 zu sperren.

Die Gassteuervorrichtung 10 ist pneumatisch und/oder elektronisch ausgeführt und entsprechend einer Anwendervorgabe, über eine Anwenderschnittstelle, oder automatisch, wenn die Schlauchsysteme aufgrund technischer Kennungen von einem Erkennungssystem des Beatmungsgerätes erkannt werden, eine Voreinstellung des Beatmungsgerätes für die Verwendung der alternativen Schlauchsysteme 33 vornimmt.

Die Gassteuervorrichtung 10 umfasst beispielsweise zumindest das Rückschlagventil 28a, den Bypass und das Ventil 13a. Die Gassteuervorrichtung 10 umfasst alle Ventile und Schalteinrichtungen und Atemgasquellen.

Die Gassteuervorrichtung 10 umfasst beispielsweise Geräteausgang 30, 38, Anschluss 40, 41 und zumindest eine Schalteinrichtung 46, 47 und ist umbaufrei und adapterlos eingerichtet und ausgebildet sind, sodass ein Wechsel der Schlauchsysteme 33 ohne Adapter oder ohne Werkzeuge möglich ist.

Insbesondere verbleiben der Geräteeingang und der Geräteausgang umbaufrei am Beatmungsgerät. Für die Verwendung der unterschiedlichen Schlauchsysteme sind bei einem Wechsel derselben Adapter für den Geräteeingang und den Geräteausgang nicht notwendig. Insbesondere müssen Geräteeingang oder Geräteausgang oder die Stutzen oder Anschlüse nicht mit Stopfen oder Kappen verschlossen werden.

### Bezugszeichenliste

- 10: Gassteuerungsvorrichtung
- 11: erster Gaskanal
- 12: zweiter Gaskanal
- 13a, b, c, d: Schalteinrichtung
- 14: Öffnung
- 15: Dichtmembran
- 16: Verschlusskappe der Öffnung
- 17: Gewicht
- 18: Fortsätze der Verschlusskappe
- 19: Rückschlagventil
- 20: Anschluss
- 21: Umgehungskanal
- 22: Rand der Öffnung/ Erhebung
- 23: Begrenzung der Dichtmembran
- 24: Rand des Umgehungskanals
- 25: Gebläse
- 26: Beatmungsgerät
- 27: Bypass
- 28a, b: Rückschlagventil
- 29a, b,: erster Atemgasweg, inspiratorischer Atemgasweg
- 29c, d: zweiter Atemgasweg, exspiratorischer Atemgasweg
- 30a, b: Geräteausgang
- 31a, b: Geräteeingang
- 32a, b, c, d: Flussmesseinrichtung
- 33: Schlauchsystem
- 33a: Einschlauchsystem mit Patientenventil 39
- 33b: Einschlauchsystem mit Leckagesystem
- 33c: Zweischlauschsystem
- 34: erster Zweig Zweischlauschsystem
- 35: zweiter Zweig Zweischlauschsystem
- 36: Patienten Interface
- 37a, b, c: exspiratorischer Geräteausgang
- 38: exspiratorischer Geräteeingang
- 39: Patientenventil
- 40: Drucksensor
- 41: Patientenventilanschluss
- 42: Temperatürfühler
- 43: internes Patientenventil
- 44: exspiratorischer Block
- 45: inspiratorischer Block
- 45a: Druckseite
- 45b: Saugseite
- 46: Ventil

## Patentansprüche

1. Beatmungsgerät zur Verwendung mit alternativen Schlauchsystemen (33, 33a, 33b, 33c), wobei das Beatmungsgerät umfasst:
zumindest eine Atemgasquelle (25);
zumindest einen Geräteausgang (30, 30a) und einen Geräteeingang (31, 31a);
zumindest einen Atemgasweg (27, 29, 29a, 29b), der einen ersten Atemgasweg (29a) und einen Bypass (27) umfasst, wobei sich der erste Atemgasweg (29a) zwischen dem Geräteeingang (31a) und dem Geräteausgang (30a) erstreckt; und
eine Gassteuerungsvorrichtung (10) zum Voreinstellen des Beatmungsgerätes für die Verwendung der alternativen Schlauchsysteme (33, 33a, 33b, 33c) durch Schalten zumindest einer Schalteinrichtung (13, 13a, 46, 47), wobei die Gassteuerungsvorrichtung (10) ausgebildet ist, um für die Verwendung eines der Schlauchsysteme, das ein Leckagesystem (33b) ist, den Atemgasweg (27) für einen Atemgasstrom zu öffnen;
wobei der erste Atemgasweg (29a) einen Atemgasantrieb (25a) und die Gassteuerungsvorrichtung (10) mit dem Bypass (27), der Schalteinrichtung (13a) und einem Rückschlagventil (28a) umfasst, wobei der Atemgasantrieb (25a) ausgebildet ist, um eine inspiratorische Atemgasströmung vom Geräteeingang (31a) durch das Rückschlagventil (28a) zum Geräteausgang (30a) zu führen, wobei das Rückschlagventil (28a) angeordnet und ausgebildet ist, um eine Atemgasströmung in Richtung des Geräteeingangs (31a) zu verhindern, wobei der Bypass (27) angeordnet und ausgebildet ist, um eine Atemgasströmung zum Umgehen des Rückschlagventils (28a) zu ermöglichen, und wobei die Schalteinrichtung (13a) angeordnet und ausgebildet ist, um eine Atemgasströmung durch den Bypass (27) zum Umgehen des Rückschlagventils (28a) zumindest zeitweise zu ermöglichen oder zu unterbrechen;
**dadurch gekennzeichnet, dass** die Schalteinrichtung (13a) als ein erstes Ventil (13a) ausgeführt ist und eine weitere Schalteinrichtung (47) als ein zweites Ventil (47) ausgeführt ist, wobei das zweite Ventil (47) ausgebildet ist, um zumindest zeitweise einen Rückatemdruck einer Exspiration eines Patienten als einen Steuerdruck auf das erste Ventil (13a) zu schalten.

2. Beatmungsgerät nach Anspruch 1,
wobei die Gassteuerungsvorrichtung (10) ausgebildet ist, um die Schalteinrichtung (13a, 46, 47) zu aktivieren, sodass der Bypass (27) für den Atemgasstrom geöffnet wird und ein Patientenventilanschluss (41) geschlossen wird.

3. Beatmungsgerät nach Anspruch 2,
wobei die Gassteuerungsvorrichtung (10) ausgebildet ist, um für die Verwendung eines der Schlauchsysteme, das ein Ventilsystem (33a) mit einem Patientenventil (39) ist, die Schalteinrichtung (13a, 46, 47) zu aktivieren, sodass der Atemgasstrom durch den Bypass (27) gesperrt wird und der Patientenventilanschluss (41) geöffnet wird, wodurch ein Steuerdruck auf das Patientenventil (39) geschaltet wird.

4. Beatmungsgerät nach Anspruch 2 oder 3,
wobei die Gassteuerungsvorrichtung (10) ausgebildet ist, um für die Verwendung eines der Schlauchsysteme, das ein Zweischlauchsystem ist, den Bypass (27) und den Patientenventilanschluss (41) zu sperren.

5. Beatmungsgerät nach einem der Ansprüche 2 bis 4,
wobei die Gassteuerungsvorrichtung (10) ausgebildet ist, um die Schalteinrichtung (13a, 46, 47) zu aktivieren, sodass der Atemgasstrom durch den Bypass (27) und der Patientenventilanschluss (41) gesperrt werden.

6. Beatmungsgerät nach einem der Ansprüche 2 bis 5,
wobei der Atemgasweg (27, 29), der Geräteausgang (30, 38), der Patientenventilanschluss (41) und die zumindest eine Schalteinrichtung (46, 47) umbaufrei und adapterlos ausgebildet sind, sodass ein Wechsel der Schlauchsysteme (33, 33a, 33b, 33c) ohne Adapter oder ohne Werkzeuge möglich ist.

7. Beatmungsgerät nach einem der vorhergehenden Ansprüche,
wobei die Gassteuerungsvorrichtung (10) pneumatisch und/oder elektronisch ausgeführt ist und ausgebildet ist, um das Beatmungsgerät für die Verwendung der alternativen Schlauchsysteme (33, 33a, 33b, 33c) entsprechend einer Anwendervorgabe über eine Anwenderschnittstelle oder automatisch, wenn das jeweilige Schlauchsystem aufgrund technischer Kennungen von einem Erkennungssystem des Beatmungsgerätes erkannt wird, voreinzustellen.

8. Beatmungsgerät nach einem der vorhergehenden Ansprüche,
wobei die Schalteinrichtung (46) als ein Verschlussventil ausgeführt ist, das ausgebildet ist, um den Steuerdruck, der aus der Atemgasquelle (25) kommt, auf einen Druckstutzen (41) zu schalten, wenn dieser mit einem Patientenventil (39) eines Einschlauchsystems verbunden ist.

9. Beatmungsgerät nach einem der vorhergehenden Ansprüche,
wobei die Schalteinrichtung (13) im Bypass (27) angeordnet ist und der Bypass (27) vom ersten Atemgasweg (29a) vor dem Rückschlagventil (28a) abzweigt und hinter dem Rückschlagventil (28a) wieder in den ersten Atemgasweg (29a) oder in den Geräteeingang mündet.

10. Beatmungsgerät nach einem der vorhergehenden Ansprüche,
wobei der Atemgasantrieb (25a) zwischen dem Geräteeingang (31a) und dem Rückschlagventil (28a) oder zwischen dem Rückschlagventil (28a) und dem Geräteausgang (30a) angeordnet ist.

11. Beatmungsgerät nach einem der vorhergehenden Ansprüche,
wobei die Schalteinrichtung (13) im Bypass (27) angeordnet ist und der Bypass (27) zwischen dem Atemgasantrieb (25a) und dem Rückschlagventil (28a) abzweigt und zwischen dem Rückschlagventil (28a) und dem Geräteausgang (30a) wieder in den ersten Atemgasweg (29a) mündet, wobei der Atemgasantrieb (25a) zwischen dem Geräteeingang (31a) und dem Rückschlagventil (28a) angeordnet ist und über die im Bypass (27) angeordnete Schalteinrichtung (13a) Atemgas in Richtung des Geräteeingangs (31a) rückgeführt werden kann, wodurch das Rückschlagventil (28a) umgangen wird.

12. Beatmungsgerät nach einem der vorhergehenden Ansprüche,
wobei die Schalteinrichtung (13) im Bypass (27) angeordnet ist und der Bypass (27) zwischen dem Geräteeingang (31a) und dem Rückschlagventil (28a) abzweigt und zwischen dem Rückschlagventil (28a) und dem Atemgasantrieb (25a) wieder in den ersten Atemgasweg (29a) mündet, wobei der Atemgasantrieb (25a) zwischen dem Rückschlagventil (28a) und dem Geräteausgang (30a) angeordnet ist und über die im Bypass (27) angeordnete Schalteinrichtung (13a) Atemgas in Richtung des Geräteeingangs (31a) rückgeführt werden kann, nachdem es den Atemgasantrieb (25a) passiert hat.

13. Beatmungsgerät nach einem der vorhergehenden Ansprüche,
wobei eine Gasmesseinrichtung (32a) zwischen dem Bypass (27) und dem Geräteausgang (30a) angeordnet ist und ausgebildet ist, um einen Fluss oder ein Volumen oder einen Druck des Atemgases im Atemgasweg zu erfassen, wobei die Schalteinrichtung (13a) ausgebildet ist, um einen positiven endexspiratorischen Druck im Bereich von 0 bis 20 hPa, bevorzugt 0 bis 15 hPa, einzustellen.

14. Beatmungsgerät nach einem der vorhergehenden Ansprüche,
wobei das Beatmungsgerät einen exspiratorischen Atemgasweg (29c), der sich von einem exspiratorischen Geräteeingang (38) zu einem exspiratorischen Geräteausgang (37a) erstreckt, aufweist, wobei das Beatmungsgerät ferner eine Schalteinrichtung (13b) und eine Gasmesseinrichtung (32c) umfasst, wobei die Gasmesseinrichtung (32c) zwischen dem exspiratorischen Geräteausgang (37a) und der Schalteinrichtung (13b) angeordnet ist.

15. Beatmungsgerät nach Anspruch 14,
wobei eines der Schlauchsysteme (33) an den Geräteausgang (30a) montiert ist, wobei das Schlauchsystem (33) einen ersten Zweig (34) und einen zweiten Zweig (35) aufweist, wobei der erste Zweig (34) vom Geräteausgang (30a) zu einem Patienteninterface (36) führt und der zweite Zweig (35) vom Patienteninterface (36) zum exspiratorischen Geräteeingang (38) führt, wobei über den ersten Zweig (34) inspiratorisches Atemgas aus einem inspiratorischen Atemgasweg (29a) als dem ersten Atemgasweg (29a) zum Patienteninterface (36) geführt werden kann und über den zweiten Zweig (35) exspiratorisches Atemgas aus dem Patienteninterface (36) zum exspiratorischen Atemgasweg (29c) geführt werden kann.

16. Beatmungsgerät nach Anspruch 15,
wobei bei einer Blockade oder Störung des exspiratorischen Atemgasweges (29c) das exspiratorische Atemgas durch Steuern der Schalteinrichtung (13a) in den inspiratorischen Atemgasweg (29a) und über den geöffneten Bypass (27) in die Umgebung abgeführt werden kann.

17. Beatmungsgerät nach Anspruch 15 oder 16,
wobei das Beatmungsgerät den ersten inspiratorischen Atemgasweg (29a) und einen zweiten inspiratorischen Atemgasweg (29b) sowie einen ersten exspiratorischen Atemgasweg (29c) und einen separaten zweiten exspiratorischen Atemgasweg (29d) aufweist.

## Claims

1. A ventilator for use with alternative hose systems (33, 33a, 33b, 33c), wherein the ventilator comprises:
at least one respiratory gas source (25);
at least one device outlet (30, 30a) and one device inlet (31, 31a);
at least one respiratory gas path (27, 29, 29a, 29b), which comprises a first respiratory gas path (29a) and a bypass (27), wherein the first respiratory gas path (29a) extends between the device inlet (31a) and the device outlet (30a); and
a gas control device (10) for presetting the ventilator for the use of the alternative hose systems (33, 33a, 33b, 33c) by switching at least one switching apparatus (13, 13a, 46, 47), wherein the gas control device (10) is designed to open the respiratory gas path (27) for a respiratory gas flow for the use of one of the hose systems, which is a leakage system (33b);
wherein the first respiratory gas path (29a) comprises a respiratory gas drive (25a) and the gas control device (10) with the bypass (27), the switching apparatus (13a), and a check valve (28a), wherein the respiratory gas drive (25a) is designed to guide an inspiratory respiratory gas flow from the device inlet (31a) through the check valve (28a) to the device outlet (30a), wherein the check valve (28a) is arranged and designed to prevent a respiratory gas flow in the direction of the device inlet (31a), wherein the bypass (27) is arranged and designed to enable a respiratory gas flow to bypass the check valve (28a), and wherein the switching apparatus (13a) is arranged and designed to at least temporarily enable or interrupt a respiratory gas flow through the bypass (27) to bypass the check valve (28a);
**characterized in that** the switching apparatus (13a) is designed as a first valve (13a) and a further switching apparatus (47) is designed as a second valve (47), wherein the second valve (47) is designed to at least temporarily switch a return breathing pressure of an expiration of a patient to the first valve (13a) as a control pressure.

2. The ventilator according to claim 1,
wherein the gas control device (10) is designed to activate the switching apparatus (13a, 46, 47) so that the bypass (27) is opened for the respiratory gas flow and a patient valve connection (41) is closed.

3. The ventilator according to claim 2,
wherein the gas control device (10) is designed to activate the switching apparatus (13a, 46, 47) for the use of one of the hose systems, which is a valve system (33a) with a patient valve (39), so that the respiratory gas flow through the bypass (27) is blocked and the patient valve connection (41) is opened, whereby a control pressure is switched to the patient valve (39).

4. The ventilator according to claim 2 or 3,
wherein the gas control device (10) is designed to block the bypass (27) and the patient valve connection (41) for the use of one of the hose systems, which is a two-hose system.

5. The ventilator according to one of claims 2 to 4,
wherein the gas control device (10) is designed to activate the switching apparatus (13a, 46, 47) so that the respiratory gas flow through the bypass (27) and the patient valve connection (41) are blocked.

6. The ventilator according to one of claims 2 to 5,
wherein the respiratory gas path (27, 29), the device outlet (30, 38), the patient valve connection (41), and the at least one switching apparatus (46, 47) are designed to be alteration-free and adapterless so that changing the hose systems (33, 33a, 33b, 33c) is possible without an adapter or without tools.

7. The ventilator according to one of the preceding claims,
wherein the gas control device (10) is designed pneumatically and/or electronically and is designed to preset the ventilator for the use of the alternative hose systems (33, 33a, 33b, 33c) in accordance with a user specification via a user interface or automatically when the respective hose system is detected by a detection system of the ventilator due to technical identifiers.

8. The ventilator according to one of the preceding claims,
wherein the switching apparatus (46) is designed as a closure valve, which is designed to switch the control pressure that comes out of the respiratory gas source (25) to a discharge port (41) when said discharge port is connected to a patient valve (39) of a single-hose system.

9. The ventilator according to one of the preceding claims,
wherein the switching apparatus (13) is arranged in the bypass (27) and the bypass (27) branches off from the first respiratory gas path (29a) upstream of the check valve (28a) and opens again into the first respiratory gas path (29a) or into the device inlet downstream of the check valve (28a).

10. The ventilator according to one of the preceding claims,
wherein the respiratory gas drive (25a) is arranged between the device inlet (31a) and the check valve (28a) or between the check valve (28a) and the device outlet (30a).

11. The ventilator according to one of the preceding claims,
wherein the switching apparatus (13) is arranged in the bypass (27) and the bypass (27) branches off between the respiratory gas drive (25a) and the check valve (28a) and opens again into the first respiratory gas path (29a) between the check valve (28a) and the device outlet (30a), wherein the respiratory gas drive (25a) is arranged between the device inlet (31a) and the check valve (28a) and respiratory gas can be returned in the direction of the device inlet (31a) via the switching apparatus (13a) arranged in the bypass (27), whereby the check valve (28a) is bypassed.

12. The ventilator according to one of the preceding claims,
wherein the switching apparatus (13) is arranged in the bypass (27) and the bypass (27) branches off between the device inlet (31a) and the check valve (28a) and opens again into the first respiratory gas path (29a) between the check valve (28a) and the respiratory gas drive (25a), wherein the respiratory gas drive (25a) is arranged between the check valve (28a) and the device outlet (30a) and respiratory gas can be returned in the direction of the device inlet (31a) via the switching apparatus (13a) arranged in the bypass (27) after it has passed the respiratory gas drive (25a).

13. The ventilator according to one of the preceding claims,
wherein a gas measuring apparatus (32a) is arranged between the bypass (27) and the device outlet (30a) and is designed to detect a flow or a volume or a pressure of the respiratory gas in the respiratory gas path, wherein the switching apparatus (13a) is designed to set a positive end-expiratory pressure in the range from 0 to 20 hPa, preferably 0 to 15 hPa.

14. The ventilator according to one of the preceding claims,
wherein the ventilator has an expiratory respiratory gas path (29c), which extends from an expiratory device inlet (38) to an expiratory device outlet (37a), wherein the ventilator further comprises a switching apparatus (13b) and a gas measuring apparatus (32c), wherein the gas measuring apparatus (32c) is arranged between the expiratory device outlet (37a) and the switching apparatus (13b).

15. The ventilator according to claim 14,
wherein one of the hose systems (33) is mounted on the device outlet (30a), wherein the hose system (33) has a first branch (34) and a second branch (35), wherein the first branch (34) leads from the device outlet (30a) to a patient interface (36) and the second branch (35) leads from the patient interface (36) to the expiratory device inlet (38), wherein inspiratory respiratory gas from an inspiratory respiratory gas path (29a) as the first respiratory gas path (29a) can be guided via the first branch (34) to the patient interface (36) and expiratory respiratory gas from the patient interface (36) can be guided via the second branch (35) to the expiratory respiratory gas path (29c).

16. The ventilator according to claim 15,
wherein, if the expiratory respiratory gas path (29c) is blocked or disrupted, the expiratory respiratory gas can be discharged into the inspiratory respiratory gas path (29a) and via the opened bypass (27) to the environment by controlling the switching apparatus (13a).

17. The ventilator according to claim 15 or 16,
wherein the ventilator has the first inspiratory respiratory gas path (29a) and a second inspiratory respiratory gas path (29b) as well as a first expiratory respiratory gas path (29c) and a separate second expiratory respiratory gas path (29d).

## Revendications

1. Appareil de ventilation destiné à être utilisé avec des systèmes de tube alternatifs (33, 33a, 33b, 33c), l'appareil de ventilation comportant :
au moins une source de gaz respiratoire (25) ;
au moins une sortie d'appareil (30, 30a) et une entrée d'appareil (31, 31a) ;
au moins un trajet de gaz respiratoire (27, 29, 29a, 29b), lequel comporte une premier trajet de gaz respiratoire (29a) et une dérivation (27), le premier trajet de gaz respiratoire (29a) s'étendant entre l'entrée d'appareil (31a) et la sortie d'appareil (30a) ; et
un dispositif de commande de gaz (10) destiné à prérégler l'appareil de ventilation pour l'utilisation de systèmes de tube alternatifs (33, 33a, 33b, 33c) par commutation d'au moins un moyen de commutation (13, 13a, 46, 47), le dispositif de commande de gaz (10) étant conçu pour ouvrir le trajet de gaz respiratoire (27) pour un flux de gaz respiratoire pour l'utilisation de l'un des systèmes de tube, lequel est un système de fuite (33b) ;
dans lequel le premier trajet de gaz respiratoire (29a) comporte un entraînement de gaz respiratoire (25a) et le dispositif de commande de gaz (10) avec la dérivation (27), le moyen de commutation (13a) et une soupape antiretour (28a), dans lequel l'entraînement de gaz respiratoire (25a) est conçu pour guider un écoulement de gaz respiratoire inspiratoire depuis l'entrée d'appareil (31a) vers la sortie d'appareil (30a) à travers la soupape antiretour (28a), dans lequel la soupape antiretour (28a) est agencée et conçue pour empêcher un écoulement de gaz respiratoire en direction de l'entrée d'appareil (3 1a), dans lequel la dérivation (27) est agencée et conçue pour permettre un écoulement de gaz respiratoire destiné à contourner la soupape antiretour (28a), et dans lequel le moyen de commutation (13a) est agencé et conçu pour permettre ou interrompre au moins temporairement un écoulement de gaz respiratoire à travers la dérivation (27) pour contourner la soupape antiretour (28a) ;
**caractérisé en ce que** le moyen de commutation (13a) est réalisé comme une première soupape (13a) et un autre moyen de commutation (47) est réalisé comme une deuxième soupape (47), la deuxième soupape (47) étant conçue pour commuter au moins temporairement une contre-pression respiratoire d'une expiration d'un patient en tant que pression de commande sur la première soupape (13a).

2. Appareil de ventilation selon la revendication 1,
dans lequel le dispositif de commande de gaz (10) est conçu pour activer le moyen de commutation (13a, 46, 47) de manière à ouvrir la dérivation (27) soit ouverte pour le flux de gaz respiratoire et à fermer un raccord de soupape de patient (41).

3. Appareil de ventilation selon la revendication 2,
dans lequel, pour l'utilisation de l'un des systèmes de tube, lequel est un système de soupape (33a) avec une soupape de patient (39), le dispositif de commande de gaz (10) est conçu pour activer le moyen de commutation (13a, 46, 47) de manière à bloquer le flux de gaz respiratoire à travers la dérivation (27) et à ouvrir le raccord de soupape de patient (41), moyennant quoi une pression de commande est commutée sur la soupape de patient (39).

4. Appareil de ventilation selon la revendication 2 ou 3,
dans lequel le dispositif de commande de gaz (10) est conçu pour bloquer la dérivation (27) et le raccord de soupape de patient (41) pour l'utilisation de l'un des système de tube, lequel est un système à deux tubes.

5. Appareil de ventilation selon l'une des revendications 2 à 4,
dans lequel le dispositif de commande de gaz (10) est conçu pour activer le moyen de commutation (13a, 46, 47) de manière à bloquer le flux de gaz respiratoire à travers la dérivation (27) et le raccord de soupape de patient (41).

6. Appareil de ventilation selon l'une des revendications 2 à 5,
dans lequel le trajet de gaz respiratoire (27, 29), la sortie d'appareil (30, 38), le raccord de soupape de patient (41) et l'au moins un moyen de commutation (46, 47) sont conçus sans conversion et sans adaptateur, de manière à permettre un remplacement des systèmes de tube (33, 33a, 33b, 33c) sans adaptateur ou sans outils.

7. Appareil de ventilation selon l'une des revendications précédentes,
dans lequel le dispositif de commande de gaz (10) est réalisé et conçu pneumatiquement et/ou électroniquement pour prérégler l'appareil de ventilation pour l'utilisation des systèmes de tube alternatifs (33, 33a, 33b, 33c) conformément à un paramètre d'utilisateur par le biais d'une interface d'utilisateur ou automatiquement, lorsque le système de tube respectif est reconnu par un système de reconnaissance de l'appareil de ventilation sur la base d'identifiants techniques.

8. Appareil de ventilation selon l'une des revendications précédentes,
dans lequel le moyen de commutation (46) est réalisé comme une soupape de fermeture, laquelle est conçue pour commuter la pression de commande venant de la source de gaz respiratoire (25) sur une tubulure de pression (41) lorsque celle-ci est raccordée à une soupape de patient (39) d'un système à un tube.

9. Appareil de ventilation selon l'une des revendications précédentes,
dans lequel le moyen de commutation (13) est agencé dans la dérivation (27) et la dérivation (27) dévie du premier trajet de gaz respiratoire (29a) en amont de la soupape antiretour (28a) et débouche de nouveau dans le premier trajet de gaz respiratoire (29a) ou dans l'entrée d'appareil en aval de la soupape antiretour (28a).

10. Appareil de ventilation selon l'une des revendications précédentes,
dans lequel l'entraînement de gaz respiratoire (25a) est agencé entre l'entrée d'appareil (31a) et la soupape antiretour (28a) ou entre la soupape antiretour (28a) et la sortie d'appareil (30a).

11. Appareil de ventilation selon l'une des revendications précédentes,
dans lequel le moyen de commutation (13) est agencé dans la dérivation (27) et la dérivation (27) dévie entre l'entraînement de gaz respiratoire (25a) et la soupape antiretour (28a) et débouche de nouveau dans le premier trajet de gaz respiratoire (29a) entre la soupape antiretour (28a) et la sortie d'appareil (30a), dans lequel l'entraînement de gaz respiratoire (25a) est agencé entre l'entrée d'appareil (31a) et la soupape antiretour (28a) et du gaz respiratoire peut être renvoyé en direction de l'entrée d'appareil (31a) par le biais du moyen de commutation (13a) agencé dans la dérivation (27), moyennant quoi la soupape antiretour (28a) est contournée.

12. Appareil de ventilation selon l'une des revendications précédentes,
dans lequel le moyen de commutation (13) est agencé dans la dérivation (27) et la dérivation (27) dévie entre l'entrée d'appareil (31a) et la soupape antiretour (28a) et débouche de nouveau dans le premier trajet de gaz respiratoire (29a) entre la soupape antiretour (28a) et l'entraînement de gaz respiratoire (25a), dans lequel l'entraînement de gaz respiratoire (25a) est agencé entre la soupape antiretour (28a) et la sortie d'appareil (30a) et du gaz respiratoire peut être renvoyé en direction de l'entrée d'appareil (31a) par le biais du moyen de commutation (13a) agencé dans la dérivation (27), après son passage devant l'entraînement de gaz respiratoire (25a).

13. Appareil de ventilation selon l'une des revendications précédentes,
dans lequel un moyen de mesure de gaz (32a) est agencé entre la dérivation (27) et la sortie d'appareil (30a) et conçu pour détecter un flux ou un volume ou une pression du gaz respiratoire dans le trajet de gaz respiratoire, dans lequel le moyen de commutation (13a) est conçu pour régler une pression de fin d'expiration positive dans la plage de 0 à 20 hPa, de préférence de 0 à 15 hPa.

14. Appareil de ventilation selon l'une des revendications précédentes,
dans lequel l'appareil de ventilation présente un trajet de gaz respiratoire expiratoire (29c), lequel s'étend à partir d'une entrée d'appareil expiratoire (38) vers une sortie d'appareil expiratoire (37a), l'appareil de ventilation comportant en outre un moyen de commutation (13b) et un moyen de mesure de gaz (32c), le moyen de mesure de gaz (32c) étant agencé entre la sortie d'appareil expiratoire (37a) et le moyen de commutation (13b).

15. Appareil de ventilation selon la revendication 14,
dans lequel l'un des systèmes de tube (33) est monté sur la sortie d'appareil (30a), dans lequel le système de tube (33) présente une première branche (34) et une deuxième branche (35), dans lequel la première branche (34) mène de la sortie d'appareil (30a) vers une interface de patient (36) et la deuxième branche (35) mène de l'interface de patient (36) vers l'entrée d'appareil expiratoire (38), dans lequel du gaz respiratoire inspiratoire peut être guidé par le biais de la première branche (34) hors d'un trajet de gaz respiratoire inspiratoire (29a) en tant que premier trajet de gaz respiratoire (29a) vers l'interface de patient (36) et du gaz respiratoire expiratoire peut être guidé par le biais de la deuxième branche (35) hors de l'interface de patient (36) vers le trajet de gaz respiratoire expiratoire (29c).

16. Appareil de ventilation selon la revendication 15,
dans lequel, en cas d'obstruction ou de panne du trajet de gaz respiratoire expiratoire (29c), le gaz respiratoire expiratoire peut être évacué vers le trajet de gaz respiratoire inspiratoire (29a) et vers l'environnement par le biais de la dérivation (27) ouverte, par commande du moyen de commutation (13a).

17. Appareil de ventilation selon la revendication 15 ou 16,
dans lequel l'appareil de ventilation présente le premier trajet de gaz respiratoire inspiratoire (29a) et un deuxième trajet de gaz respiratoire inspiratoire (29b) ainsi qu'un premier trajet de gaz respiratoire expiratoire (29c) et un deuxième trajet de gaz respiratoire expiratoire séparé (29d).
